# Europäisches Patentamt
## European Patent Office
### Office européen des brevets

(11) Veröffentlichungsnummer : **0 471 647 A2**

(12) **EUROPÄISCHE PATENTANMELDUNG**

(21) Anmeldenummer : **91810617.0**

(22) Anmeldetag : **07.08.91**

(51) Int. Cl.⁵ : **C12N 15/75**, C12N 1/21, C12N 15/87, // (C12N1/21, C12R1:07), (C12N1/21, C12R1:085)

(30) Priorität : **16.08.90 CH 2660/90**

(43) Veröffentlichungstag der Anmeldung :
**19.02.92 Patentblatt 92/08**

(84) Benannte Vertragsstaaten :
**AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Schurter, Walter, Dr.**
**Burgfeldermattweg 57**
**CH-4123 Allschwil (CH)**
Erfinder : **Reinhard, Christina**
**Bruckmühlenweg 10**
**W-7815 Kirchzarten (DE)**
Erfinder : **Mathé, Danièle**
**39, Rud d'Istein**
**F-68870 Bartenheim la Chaussée (FR)**

(54) **Restriktionsdefiziente Mutante.**

(57)   Gegenstand der vorliegenden Erfindung ist eine Mutante von *Bacillus thuringiensis* bzw. *B. cereus*, die eine partielle Defiziens der inhärent im unmutierten Ausgangsstamm vorliegenden Restriktionsbarrieren aufweist und daher bei Verwendung von Vektor-DNA aus einem heterologen Zwischenwirt, die natürlicherweise einer Restriktion in *B. thuringiensis* und/oder *B. cereus* unterliegt, im Vergleich zu besagtem, nicht-mutierten Ausgangsstamm signifikant besser transformierbar ist.
Ebenso umfasst sind Verfahren zur Herstellung besagter Mutanten, die auf einer gezielten Anreicherung von Spontanmutanten basieren sowie Verfahren zur Klonierung von Genen oder sonstigen DNA Sequenzen unter Verwendung von besagten restriktionsdefizienten Mutanten von *Bacillus thuringiensis* und/oder *Bacillus cereus*.

EP 0 471 647 A2

Die vorliegende Erfindung betrifft partiell restriktionsdefiziente Mutanten von *Bacillus thuringiensis* bzw. *B-.cereus* sowie Verfahren zur Herstellung besagter Mutanten. Ebenso umfasst sind Verfahren zur Überwindung von Restriktionsbarrieren bei *Bacillus thuringiensis* und/oder *Bacillus cereus* unter Verwendung besagter restriktionsdefizienter Mutanten.

Restriktions/Modifikationssysteme sind bei Mikroorganismen weit verbreitet und seit langem bekannt. Mittlerweile sind in der wissenschaftlichen Literatur mehr als 600 Restriktionsenzyme und ca. 100 der dazugehörigen Methylasen beschrieben [Kessler & Höltke (1986)].

Die weitaus grösste Anzahl der bisher beschriebenen Restriktions/Modifikationssysteme gehören den sog. Klasse II-Systemen an. Diese Klasse II-Systeme sind durch eine nur geringe Komplexität gekennzeichnet und verwenden dementsprechend relativ einfache Proteine. Ein Klasse II Restriktionsenzym beispielsweise, ist in der Lage eine bestimmte DNA-Sequenz zu erkennen und zu schneiden, sofern diese nicht zuvor bereits durch die zugehörige Methylase spezifisch methyliert wurde. Beide Enzyme benötigen kein ATP. Die für diese Enzyme [Methylasen] kodierenden Gene liegen häufig eng gekoppelt nebeneinander auf dem bakteriellen Genom.

Die wenigen bekannten Restriktions/Modifikationssysteme der komplexeren Klassen I und III stammen von *Escherichia coli*, *Samonella* und *Haemophilus* [Kessler & Höltke (1986)].

Zusätzlich zu diesen "klassischen" Systemen sind in den letzten Jahren zuerst in *E. coli*, dann aber auch in anderen Spezies Restriktionsenzyme gefunden worden, die, in Umkehrung der Verhältnisse bei den Klasse II-Systemen, eine DNA-Sequenz nur dann erkennen und schneiden, wenn diese spezifisch methyliert ist [Raleigh & Wilson (1986); Sladek *et al* (1986) und MacNeil (1988)].

Die klassischen Restriktionsenzyme der Klasse II stellen eines der wichtigsten Hilfsmittel im Rahmen der rDNA Technologie dar und bilden somit eine der essentiellen Grundlagen für die moderne Molekulargenetik. Sie finden in erster Linie Anwendung bei der Konstruktion rekombinanter DNA Moleküle.

Ihre natürliche Funktion in der Zelle dagegen betrifft die Abwehr von unerwünschter Fremd-DNA, wie z.B. viraler oder bakterieller DNA. Aufgrund dieser natürlichen Funktion, die auf dem Abbau der in die Zelle eingedrungenen Fremd-DNA beruht, muss man beim Arbeiten mit rekombinanter DNA mit zum Teil drastisch reduzierten Transformationsfrequenzen rechnen. Man spricht in diesem Zusammenhang vom Vorliegen sogenannter Restriktionsbarrieren [Matsushima *et al* (1987); Miller *et al* (1988) und McDonald & Burke, (1984)]. Dabei können bereits geringfügige Restriktionsbarrieren ausreichen um die Konstruktion einer representativen Genbank weitgehend zu verhindern, da der Restriktionseffekt mit zunehmender Grösse des rekombinanten Plasmids zunimmt. Vollständig intakte Gene lassen sich daher beim vorliegen einer Restriktion nur unter grossen Schwierigkeiten isolieren. Im Falle von *B. subtilis* beispielsweise konnte gezeigt werden, dass das in diesem Stamm vorkommende Restriktionsenzym BsuM [Uozumi *et al* (1977); Hoshino *et al* (1980) und Bron *et al* (1988)] eine der Hauptursachen für die strukturelle Instabilität von rekombinanten Plasmiden in diesem Organismus darstellt [Haima *et al* (1987)].

Die Ueberwindung der von den Restriktionsenzymen der Klasse II verursachten Restriktionsbarrieren ist somit eine der Hauptaufgaben, die es im Rahmen der Molekulargenetik zu lösen galt und immer noch zu lösen gilt. Dies kann heute auf mehrere Arten erreicht werden.

Ein möglicher Ansatz betrifft die Verwendung nicht-restringierender Zwischenwirte, von denen die DNA ohne Restriktion in den eigentlichen Wirt übertragen werden kann. Dieser Ansatz eignet sich beispielsweise für Methylierungs-spezifische Restriktionssysteme. Für den Zwischenwirt kann z.B. eine nicht methylierende Mutante von *E. coli* oder ein natürlicherweise Methylierungs-defizienter Stamm verwendet werden [Mac Neil ( 1988); Europäisches Patent Nr. 0 341 776 A2]. Dieser Ansatz ist jedoch eher umständlich und hat zusätzlich den Nachteil, dass diese Methylierungs-defizienten *E. coli*-Stämme meist nur schlecht transformierbar sind und keineswegs ideale Wirtsstämme darstellen.

In einem weiteren Ansatz werden hitzelabile Restriktionsenzyme *in vivo* temporär inaktiviert [Bailey & Winstanley (1986); Engel (1987)]. Die Grenzen dieses Ansatzes sind durch die relativen Hitzeempfindlichkeiten von Wirt und Restriktionsenzymen gegeben.

Eine weitere Möglichkeit zur Überwindung bestehender Restriktionsbarrieren besteht in der spezifischen Methylierung der verwendeten DNA. Ist die für ein bestimmtes Restriktionssystem spezifische Methylase bekannt und gereinigt, so kann die DNA vor der Transformation spezifisch *in vitro* methyliert und dadurch vor Restriktion geschützt werden (Vehmaanperä, 1988). Ist gar das entsprechende Methylase-Gen kloniert, so ist es prinzipiell möglich, die zu transformierende DNA direkt in einem geeigneten Wirt *in vivo* zu methylieren.

Die für den Genetiker wohl befriediegenste und damit bevorzugte Lösung besteht jedoch in der Isolierung von Mutanten, welche die für die Restriktionsbarrieren verantwortlichen Restriktionsenzyme nicht länger synthetisieren. Die meisten der als Wirtsstämme verwendeten *E. coli* K-Stämme tragen zum Beispiel eine Mutation im Gen für EcoK, einem Enzym der Klasse I. Da DNA von höheren Eukaryonten meist stak methyliert ist, wurden in letzter Zeit auch *E. coli*-Stämme konstruiert, die zusätzlich Mutationen für die Methylierungs-spezifischen

2

Restriktionssysteme mcrA mcrB und mrr tragen [Kretz *et al* (1989)]. Schwierig gestaltet sich dieser Ansatz in Stämmen, die eine grössere Anzahl von Restriktionssystemen aufweisen, wie z.B. *Streptomyces fradiae* [Matsushima *et al* (1987)].

Restriktionsbarrieren sind jedoch nicht auf *E.coli* beschränkt, sondern finden sich auch bei einer Vielzahl anderer Mikroorganismen, die für Klonierungsexperimente verwendet werden.

Bei *B. thuringiensis* bzw. *B. cereus* sind Restriktions/Modifikationssysteme bisher noch recht wenig bekannt, da diese *Bacillus* Spezies erst seit relativ kurzer Zeit effizient transformiert werden können (Schurter *et al.* 1989) und daher das Interesse zur Untersuchung von Restriktions/Modifikationssystemen bei diesen Organismen bisher nur gering war. Entsprechend wurden Restriktionsbarrieren im Zusammenhang mit *B. thuringiensis* bzw. *B. cereus* bisher auch nur beiläufig erwähnt. Azizbekjan *et al* (1983) beispielsweise beschreiben ein AvaII-Isochizomer von *B. thuringiensis* var. *israelensis*.

Mit der neu geschaffenen Möglichkeit zur effizienten Transformation von *B. thuringiensis* und/oder *B. cereus* und damit zur Verwendung dieser Organismen als Klonierungsvehikel jedoch, wurden diese Restriktionsbarrieren auch bei *B. thuringiensis* bzw. *B. cereus* zum Problem.

Wird beispielsweise zur Elektroporation von *B. thuringiensis* Stamm HD1cryB [Stahly *et al* (1978)], einem Kristallkörper-freien Derivat von *B. thuringiensis* var. *kurstaki* HD1, ein Shuttlevektor verwendet, der sowohl in *B. thuringiensis* und *B. subtilis* als auch in *E. coli* replizieren kann, so hängt die erreichte Transformationsfrequenz in erster Linie davon ab, aus welchem Stamm die Plasmid DNA isoliert wurde. Die jeweils erzielten absoluten Werte können dabei zwar variieren, die relativen Frequenzen bleiben jedoch weitgehend konstant.

Ein repräsentatives Beispiel für diese Unterschiede in den Transformationsfrequenzen ist in Tabelle 1 wiedergegeben. Die Ergebnisse zeigen, dass sich beispielsweise der 'shuttle'Vektor pHY300PLK mindestens um den Faktor $10^3$ schlechter transformieren lässt, wenn das Plasmid aus einem *E. coli* Wirt anstelle eines *B. thuringiensis* Wirtes isoliert wird. Handelt es sich bei dem Ursprungsstamm um *B. subtilis*, so ist die Transformationsfrequenz ca. um den Faktor 10 erniedrigt. Wird dagegen die Plasmid DNA aus dem *B. thuringiensis* Stamm HD1cryB reisoliert, so lässt sie sich wieder mit hoher Frequenz in HD1cryB zurücktransformieren.

Dieser Restriktionseffekt erfährt noch eine Steigerung, wenn man in den Shuttlevektor zusätzliche DNA einkloniert, z.B. in Form eines Protoxingens. Man findet in diesem Fall die Restriktionsbarriere bei Transformation in einen *B. thuringiensis* Wirt nochmals deutlich erhöht [siehe Tab. 4, pXI204, pXI93]. Der Grund dafür dürfte darin zu sehen sein, dass die zusätzlich in den Vektor integrierte DNA weitere Restriktionsschnittstellen aufweist, welche eine zusätzliche Angriffsfläche für die postulierten Wirts-spezifischen [HD1cryB] Enzyme darstellen. Diese Restriktionsbarrieren können einen Wert von ca. $10^4 - 10^5$ x erreichen, was die Wahrscheinlichkeit einer erfolgreichen Transformation von HD1cryB unter Verwendung dieser Shuttlevektoren sehr gering werden lässt.

Auch in diesem Fall sind die besagten Plasmide nur dann "normal" in HD1cryB transformierbar, wenn sie zuvor aus HD1cryB isoliert wurden. Sehr oft ist es aber wünschenswert eine Klonierung von DNA in einem heterologen Zwischenwirt durchzuführen, der jedoch in vielen Fällen in Bezug auf das in *B. thuringiensis* und/oder *B. cereus* vorliegende Restriktionssystem nicht oder nur wenig kompatibel ist. Eine Transformation von *B. thuringiensis* und/oder *B. cereus* mit Vektor-DNA, die zuvor aus einem solchen heterologen, nicht-kompatiblen Zwischenwirt isoliert wurde, führt dann in der Folge oftmals zu wenig befriedigenden Ergebnissen.

Die Aufgabe, die es im Rahmen der vorliegenden Erfindung zu lösen galt bestand somit in erster Linie darin, Restriktionsbarrieren in *B. thuringiensis* und/oder *B. cereus* zu identifizieren und zu charakterisieren sowie Methoden zur Überwindung dieser Barrieren zu entwickeln. Diese Aufgabe konnte jetzt im Rahmen dieser Erfindung überraschenderweise dadurch gelöst werden, dass eine partiell Restriktions-defiziente Mutante von *B. thuringiensis* zur Verfügung gestellt wird, wie aus der nachfolgenden, detaillierten Beschreibung im einzelnen zu entnehmen ist.

Die vorliegende Erfindung betrifft somit in erster Linie eine Mutante von *B. thuringiensis* und/oder *B. cereus*, die eine partielle Defizienz der inhärent im unmutierten Ausgangsstamm vorliegenden Restriktionsbarrieren aufweist und daher bei Verwendung von Vektor-DNA aus einem heterologen Zwischenwirt, die natürlicherweise einer Restriktion in *B. thuringiensis* und/oder *B. cereus* unterliegt, im Vergleich zu besagtem, nichtmutierten Ausgangsstamm signifikant besser transformierbar ist.

Unter einem heterologen Zwischenwirt soll im Rahmen dieser Erfindung ein Wirtsorganismus verstanden werden, der für die Klonierung von Vektor-DNA geeignet ist und der zumindest nicht mit demjenigen *B. thuringiensis* und/oder *B. cereus* Stamm identisch ist, aus dem die zu klonierde DNA ursprünglich isoliert wurde.

Insbesondere betrifft die Erfindung eine partiell restriktionsdefiziente Mutante von *B. thuringiensis* bzw. *B. cereus*, die, bei Verwendung von Shuttlevektoren aus einem nichtkompatiblen Zwischenwirt wie beispielsweise *E. coli* und/oder *B. subtilis*, im Vergleich zum nicht-mutierten Ausgangsstamm signifikant, vorzugsweise aber um den Faktor $\geq 10^2$ und ganz besonders bevorzugt um den Faktor $10^2 - 10^4$ besser transformierbar ist.

Unter einem nicht-kompatiblen Zwischenwirt soll im Rahmen dieser Erfindung ein Wirtsorganismus verstanden werden, der keine Mechanismen entwickelt hat um seine DNA nach Transformation in einen *B. thuringiensis* bzw. *B. cereus* Stamm, welcher ein im Rahmen der vorliegenden Erfindung offenbartes Restriktionssystem aufweist, effektiv vor einer Restriktion zu schützen.

Eine besonders bevorzugte Ausführngsform der vorliegenden Erfindung ist eine partiell restriktionsdefiziente Mutante des *B. thuringiensis* Stammes HD1cryB, die, bei Verwendung von Shuttlevektoren aus *E. coli* und/oder *B. subtilis*, eine im Vergleich zum nicht-mutierten Ausgangsstamm um den Faktor $\geqq 10^2$, insbesondere aber um den Faktor $10^2$- $10^4$ bessere Transformierbarkeit aufweist, abhängig vom jeweils verwendeten Transformationsvektor.

Ganz besonders bevorzugt im Rahmen der vorliegenden Erfindung ist die partiell restriktionsdefiziente Mutante *B. thuringiensis* var. *kurstaki* HD1cryB Res9, die durch Spontanmutation aus dem *B.t.* Stamm HD1 cryB erhältlich ist und mit Hilfe an sich bekannter Anreicherungsverfahren gewonnen werden kann sowie Mutanten und Varianten davon, die sich unmittelbar von diesem Stamm ableiten und die noch die charakteristischen restriktionsvermindernden Eigenschaften des Ausgangsstammes aufweisen.

Ebenso umfasst von der vorliegenden Erfindung sind die Verfahren zur Herstellung besagter restriktionsdefizienter Mutanten.

Bevorzugt ist dabei ein Verfahren, das im wesentlichen dadrch gekennzeichnet ist, dass man Spontanmutanten mit verminderter Restriktionsbarriere anreichert, indem man mehrere Transformations- und Selektionsrunden hintereinanderschaltet, wobei man für die Transformation vorzugsweise Shuttlevektoren verwendet, die jeweils für unterschiedliche Selektionsmarker kodieren und die eine ausreichend hohe Transformationsrate, vorzugsweise eine Transformationsrate von $10^6$ bis $10^8$ Transformanten, garantieren und diejenigen Mutanten selektiert, die neben einer erniedrigten Restriktionsbarriere nach wie vor eine hohe Transformationseffizienz aufweisen.

Einen weiteren Gegenstand dieser Erfindung bilden Verfahren zur Reduktion von Restriktionsbarrieren bei *B. thuringiensis* und/oder *B. cereus* unter Verwendung restriktionsnegativer Mutanten, insbesondere aber unter Verwendung der restriktionsnegativen Mutanten *B. thuringiensis* HD1cryB Res9.

Ebenfalls umfasst von der Erfindung ist ein Verfahren zur Reduktion von Restriktionsbarrieren bei *B. thuringiensis* und/oder *B. cereus*, dass sich dadurch kennzeichnet, dass man eine restriktions-negative Mutante, insbesondere aber die restriktions-negative Mutante *B. thuringiensis* HD1cryB Res9, in Kombination mit einer spezifischen Methylase verwendet, die unter Methylierung der eingeschleusten DNA diese vor Verdau durch *B.thuringiensis*- bzw. *B. cereus*-eigene Restriktionsenzyme schützt und somit die Effizienz des Verfahrens weiter erhöht.

Im Rahmen der vorliegenden Erfindung soll von einer restriktionsnegativen oder -defizienten Mutanten definitionsgemäss dann gesprochen werden, wenn diese durch einen Shuttlevektor, der aus *E. coli* isoliert wurde, um einen Faktor $\geqq 10^2$ besser transformierbar ist im Vergleich zum unmutierten Ausgangsstamm. Spontantmutationen treten abei bei *B. thuringiensis* bzw. *B. cereus* meist recht selten ($10^{-6}$-$10^{-8}$) auf, sodass diese seltenen Mutanten in einem der bekannten Anreicherungsverfahren zunächst angereichert werden müssen. Dies kann vorzugsweise dadurch erreicht werden, dass man mehrere, vorzugsweise aber 1 bis 8 Runden bestehend aus Transformation und Selektion durchläuft, wobei am Ende jeder Runde die jeweils erfolgverssprechensten Transformanten ausgelesen und in der nächsten Transformations/Selektions-Runde wieder eingesetzt werden. Ist eine dieser seltenen Mutanten erst einmal transformiert, so kann in jeder neuen Runde bestehend aus Transformation/Selektion mit einer Anreicherung um einen Faktor $10^2$ - $10^3$ gerechnet werden, d.h. bereits nach wenigen Runden sollte die Zellpopulation mehrheitlich aus mutierten Zellen bestehen.

Da Spontanmutanten selten sind ($10^{-6}$ - $10^{-8}$) und daher die Wahrscheinlichkeit, dass eine dieser Mutanten transformiert wird, entsprechend gering ist, müssen die Transformationsbedingungen so gewählt werden, dass hohe Transformationsraten, vorzugsweise aber Transformationsraten von mindestens $10^6$-$10^8$ erreicht werden. Dies gilt insbesondere für die ersten beiden Transformationsrunden. Diese positiven Transformanten können dann in weiteren Transformations/Selektions-Runden angereichert, identifiziert und schliesslich isoliert werden.

Die besondere Schwierigkeit bei der Isolation Restriktions-defizienter Mutanten liegt darin, das Auftreten unerwünschter Mutanten zu vermeiden.

So kann beispielsweise die Anreicherung von spontanen, meist chromosomal kodierten Resistenzmutanten dadurch vermieden oder aber zumindest so gering wie möglich gehalten werden, dass man in jeder neuen Transformations/Selektions-Runde vorzugsweise Plasmide mit unterschiedlichen Selektionsmarkern verwendet.

Zur Anreicherung restriktionsdefizienter Mutanten werden daher vorzugsweise effizient transformierbare Vektoren eingesetzt, die eine gezielte und selektive Auswahl der positiven Transformanten erlauben. Geeignete Vektoren enthalten vorzugsweise ein oder mehrere Markergene, welche der Wirtszelle eine Eigenschaft verleihen, durch die man die mit dem Vektor transformierten Zellen leicht erkennen und in der Folge selektionieren kann. Bevorzugt sind Markergene, die eine Antibiotikaresistenz kodieren. Einige Beispiele geeigneter Resistenzgene sind insbesondere solche, welche die Antibiotika Ampicillin, Chloramphenicol, Erythromycin, Tetrazyklin, Hygromycin, G418 , Kanamycin, Bleomycin, Neomycin oder Thiostrepton kodieren.

Weiterhin bevorzugt sind Markergene, die Enzyme kodieren, für die ein chromogenes Substrat vorhanden ist. Beispiele solcher Markergene sind z.b. das lacZ-Gen [Chromogenes Substrat: X-gal → 5-Brom-4-chlor-3-indolyl-ß-D-galaktosid]; das xylE-Gen [Chromogenes Substrat: Catechol]; oder das luxAluxB-Operon, das mit langkettigen Aldehyden als Substrat [z.B. n-Decanal] eine Lichtemission erzeugt.

Die transformierten Kolonien können dann sehr einfach anhand einer spezifischen Farbreaktion nachgewiesen werden.

Ebenfalls geeignet für die Verwendung in dem erfindungsgemässen Verfahren sind Gene, die eine Resistenz gegen Schwermetalle wie z.B. Quecksilber vermitteln.

Besonders bevorzugt im Rahmen dieser Erfindung ist eine Serie von miteinander kompatiblen Shuttlevektoren, die jeweils unterschiedliche Marker kodieren und vorzugsweise in *B. thuringiensis* und/oder *B. cereus* eine gewisse Instabilität aufweisen. Aufgrund dieser Instabilität können dann später von potentiell restriktionsnegativen Mutanten relativ einfach Plasmid-freie Derivate hergestellt werden.

Diese Vektoren können darüberhinaus im Rahmen der Mutantenanreicherung aufgrund ihrer spezifischen Eigenschaft mehr als einmal zur Transformation verwendet werden.

Eine weitere Voraussetzung für das Auffinden geeigneter Mutanten sind anfänglich hohe Transformationsraten, um sicherzustellen, dass auch eine dieser selten auftretenden Mutanten mit guter Wahrscheinlichkeit mittransformiert wird. Besonders bevorzugt sind anfängliche Transformationsraten mit mindestens $10^6$- $10^8$ oder mehr Transformanten.

Die Transformation von *B. thuringiensis* und/oder *B. cereus* mit für die Transformantenselektion geeigneten Shuttlevektoren wird vorzugsweise mit Hilfe der Elektroporation durchgeführt, wie sie z.B. bei Schurter *et al*, 1989 oder in EP-A 342,633 beschrieben ist.

In einer spezifischen und im Rahmen dieser Erfindung bevorzugten Ausführungsform werden die *B. thuringiensis*-und/oder *B. cereus*-Zellen zunächst in einem geeigneten Nährmedium bei ausreichender Belüftung und bei einer geeigneten Temperatur, vorzugsweise von 20°C bis 35°C, inkubiert, bis eine optische Dichte $(OD_{550})$ von 0.1 bis 1.0 erreicht ist. Das Alter der für die Elektroporation vorgesehenen *Bacillus*-Kulturen hat einen deutlichen Einfluss auf die Transformationsfrequenz. Besonders bevorzugt ist daher eine optische Dichte der *Bacillus*-Kulturen von 0.1 bis 0.3, insbesondere aber von 0.2. Es sei jedoch darauf hingewiesen, dass sich auch mit *Bacillus*-Kulturen aus anderen Wachstumsphasen, insbesondere auch mit Übermachtkulturen gute Transformationsfrequenzen erzielen lassen.

Als Ausgangsmaterial verwendet man in der Regel frische Zellen oder Sporen, es kann aber ebensogut auch auf tiefgefrorenes Zellmaterial zurückgegriffen werden. Dabei handelt es sich vorzugsweise um Zellsuspensionen von *B. thuringiensis* und/oder *B. cereus* Zellen in geeigneten Flüssigmedien, welchen vorteilhafterweise ein bestimmter Anteil eines 'Frostschutzmittels' zugesetzt wird. Geignete Frostschutzmittel sind in erster Linie Gemische von osmotisch aktiven Komponenten und DMSO in Wasser oder einer geeigneten Pufferlösung. Weitere geeignete Komponenten, die für eine Verwendung in Frostschutzmittellösungen in Frage kommen, umfassen Zucker, mehrwertige Alkohole,wie z.B. Glycerin, Zuckeralkohole, Aminosäuren und Polymere, wie z.B. Polyethylenglykol.

Geht man von *B. thuringiensis*-Sporen aus, so werden diese zunächst in einem geeigneten Medium inokuliert und über Nacht bei einer geeigneten Temperatur, vorzugsweise von 25°C bis 28°C und ausreichender Belüftung inkubiert. Dieser Ansatz wird anschliessend verdünnt und in der oben beschriebenen Weise weiterbehandelt.

Zur Induktion der Sporulation bei *B. thuringiensis* können alle Medien verwendet werden, die eine solche Sporulation hervorrufen. Bevorzugt im Rahmen dieser Erfindung ist ein GYS-Medium nach Yousten AA und Rogoff MH, (1969).

Der Sauerstoffeintrag in das Kultivierungsmedium erfolgt in der Regel durch Bewegen der Kulturen, z.B. auf einer Schüttelmaschine, wobei Umdrehungsgeschwindigkeiten zwischen 50 Upm und 300 Upm bevorzugt sind.

Die Kultivierung von *B. thuringiensis*-Sporen sowie vegetativer Mikroorganismenzellen im Rahmen der vorliegenden Erfindung erfolgt nach bekannten, allgemein gebräuchlichen Methoden, wobei aus Gründen der Praktikabilität vorzugsweise flüssige Nährmedien verwendet werden.

Die Zusammensetzung der Nährmedien kann je nach verwendetem *B. thuringiensis*- bzw. *B. cereus* Stamm leicht variieren. Allgemein werden komplexe Medien mit wenig definierten, leicht assimilierbaren Kohlenstoff-(C-) und Stickstoff-(N-) Quellen bevorzugt, wie sie üblicherweise für die Kultivierung aerober *Bacillus*-Arten eingesetzt werden.

Ausser dem im Rahmen der vorliegenden Erfindungen bevorzugt verwendeten LB-Medium können auch alle anderen, für die Kultivierung von *B. thuringiensis* und/oder *B. cereus* geeigneten Kulturmedien verwendet werden, wie z.B. Antibiotic Medium 3, SCGY-Medium u.a. Die Aufbewahrung sporulierter *B. thuringiensis*-Kulturen erfolgt bevorzugterweise auf GYS Medien (Schrägagar) bei einer Temperatur von 4°C. [Die genaue Zusammensetzung der genannten Medien ist im Abschnitt "Medien und Pufferlösungen" wiedergegeben]

Nachdem die Zellkultur die gewünschte Zelldichte erreicht hat, werden die Zellen mit Hilfe einer Zentrifugation geerntet und in einer geeigneten Pufferlösung suspendiert, die zuvor vorzugsweise mit Eis gekühlt wird. Besonders geeignete Pufferlösungen im Rahmen dieser Erfindung sind osmotisch stabilisierte Phosphatpuffer, die als stabilisierendes Agens Zucker, wie z.B. Glucose oder Saccharose oder Zuckeralkohole, wie z.B. Mannit enthalten und auf pH-Werte von 5.0 bis 8.0 eingestellt sind. Ganz besonders bevorzugt sind Phosphatpuffer vom PBS-Typ mit einem pH-Wert von 5.0 bis 8.0, vorzugsweise von pH 5.5 bis 6.5, die Saccharose als stabilisierendes Agens in einer Konzentration von 0.1 M bis 1.0 M, vorzugsweise aber von 0.3 M bis 0.5 M enthalten.

Die Inkubationsdauer der *Bacillus*-Zellen vor und nach der Elektroporation beträgt vorzugsweise 0.1 bis 30 Minuten, insbesondere aber 10 Minuten. Die Temperatur ist in einem weiten Bereich frei wählbar. Bevorzugt ist ein Temperaturbereich von O°C bis 35°C, vorzugsweise von 2°C bis 15°C und ganz besonders bevorzugt von 4°C.

Aliquots der suspendierten Bacillus-Zellen werden anschliessend in Küvetten oder beliebige andere, geeignete Gefässe überführt und mit der zu transformierenden DNA für einen geeigneten Zeitraum, vorzugsweise für einen Zeitraum von 0.1 bis 30 Minuten, insbesondere aber von 5 bis 15 Minuten, und bei einer geeigneten Temperatur, vorzugsweise bei einer Temperatur von O°C bis 35°C, insbesondere aber bei einer Temperatur von 2°C bis 15°C und ganz besonders bevorzugt bei einer Temperatur von 4°C, gemeinsam inkubiert.

Beim Arbeiten mit tiefen Temperaturen ist es vorteilhaft bereits vorgekühlte Küvetten oder beliebige andere, geeignete und vorgekühlte Gefässe zu verwenden.

Die für eine *B. thuringiensis* oder *B. cereus* bevorzugte DNA-Konzentration liegt in einem Bereich zwischen 1 ng und 20 μg. Besonders bevorzugt ist eine DNA-Konzentration von 10 ng bis 2 μg.

Danach wird der gesamte Ansatz enthaltend *B. thuringiensis*- und/oder *B. cereus*-Zellen sowie die zu transformierende Plasmid-DNA in eine Elektroporationsapparatur eingebracht und einer Elektroporation unterzogen, d.h. kurzzeitig einem elektrischen Impuls ausgesetzt.

Elektroporationsapparaturen, die für eine Verwendung in dem erfindungsgemässen Verfahren geeignet sind, werden mittlerweile bereits von verschiedenen Herstellern angeboten, wie z.B. der Fa. Bio Rad (Richmond, CA, USA; 'Gene Pulser Apparatus'), Biotechnologies and Experimental Research, Inc. (San Diego, CA, USA; 'BTX Transfector 100'), Promega (Madison, WI, USA; 'X-Cell 2000 Electroporation System'), etc..

Selbstverständlich kann in dem erfindungsgemässen Verfahren auch jedes beliebige andere geeignete Gerät verwendet werden.

Die Kapazitätseinstellung am Kondensator beträgt vorteilhafterweise 1 μF bis 250 μF, insbesondere aber 1 μF bis 50 μF und ganz besonders bevorzugt 25 μF. Die Wahl der Ausgangsspannung ist in weiten Bereichen frei wählbar. Bevorzugt ist eine Ausgangsspannung $V_0$ von 0.2 kV bis 50 kV, insbesondere aber von 0.2 kV bis 2.5 kV und ganz besonders bevorzugt von 1.2 kV bis 1.8 kV. Der Abstand der Elektrodenplatten hängt u.a. ab von der Dimensionierung der Elektroporationsapparatur. Er beträgt vorteilhafterweise zwischen 0.1 cm und 1.0 cm, vorzugsweise zwischen 0.2 cm und 1.0 cm. Besonders bevorzugt ist ein Plattenabstand von 0.4 cm. Aus dem Abstand der Elektrodenplatten und der am Kondensator eingestellten Ausgangsspannung ergeben sich die Feldstärkewerte, die auf die Zellsuspension einwirken. Diese liegen vorteilhafterweise in einem Bereich zwischen 100 V/cm und 50'000 V/cm. Besonders bevorzugt sind Feldstärken von 100 V/cm bis 10'000 V/cm, insbesondere aber von 3'000 V/cm bis 4'500 V/cm.

Die im Rahmen des erfindugsgemässen Verfahrens bevorzugte exponentielle Abklingzeit liegt zwischen ca. 2 ms und ca. 50 ms, insbesondere aber zwischen ca. 8 ms und ca. 30 ms. Ganz besonders bevorzugt ist eine exponentielle Abklingzeit von ca. 14 ms bis ca. 20 ms.

Die Feinabstimmung der frei wählbaren Parameter, wie z.B. Kapazität, Ausgangsspannung, Plattenabstand, etc. hängt bis zu einem gewissen Grad von der Architektur der verwendeten Geräte ab und kann daher von Fall zu Fall in bestimmten Grenzen variieren. Die angegebenen Grenzwerte können daher in gewissen Fällen auch über- oder unterschritten werden sofern dies zum Erreichen optimaler Feldstärkewerte erforderlich sein sollte.

Der eigentliche Elektroporationsvorgang kann ein- bis mehrmals wiederholt werden, bis eine für das jeweilige System optimale Transformationsfrequenz erreicht ist.

Im Anschluss an die Elektroporation kann vorteilhafterweise eine Nachinkubation der behandelten *Bacillus*-Zellen durchgeführt werden, vorzugsweise für einen Zeitraum von 0.1 bis 30 Minuten, bei einer Temperatur von 0°C bis 35°C, vorzugsweise von 2°C bis 15°C. Anschliessend werden die elektroporierten Zellen mit einem geeigneten Medium verdünnt und nochmals für einen geeigneten Zeitraum, vorzugsweise von 2 bis 3 Stunden unter ausreichender Belüftung und bei einer geeigneten Temperatur, vorzugsweise von 20°C bis 35 °C, inku-

EP 0 471 647 A2

biert.

Jeweils nach Beendigung einer neuen Elektroporationsrunde mit einem der zuvor näher beschriebenen Vektoren werden die behandelten *Bacillus thuringiensis*- und/oder *B. cereus*-Zellen auf ein Selektivmedium übertragen und dort bei einer Temperatur von 10°C bis 40°C, vorzugsweise aber bei einer Temperatur von 20°C bis 35°C und ganz besonders bevorzugt bei einer Temperatur von 30°C bis 33°C inkubiert. Das Selektivmedium enthält als Selektivsubstanz vorzugsweise eine der oben genannten Antibiotika, abhängig vom verwendeten Vektor, sowie gegebenenfalls ein geeignetes Verfestigungsmittel, wie z.B. Agar, Agarose, Gelatine, etc.

Zur weiteren Beurteilung potentiell restriktions-negativer Mutanten werden zunächst Plasmid-freie Derivate der angereicherten Mutanten hergestellt. Dazu werden Einzelkolonien von den aus der jeweils letzten Transformations/ Selektionsrunde erhältlichen Anreicherungskulturen ausgelesen und auf einem geeigneten Medium ohne Selektion inkubiert. Die so erhältlichen Kulturen werden anschliessend verdünnt und auf geeigneten Festmedien, vorzugsweise auf L-Agar, ohne Selektivmarker ausplattiert. Geeignete Verdünnungen werden dann auf einem geeigneten Selektivmedium, vorzugsweise einem L-Medium, repliziert, das unterschiedliche Selektionsmarker in unterschiedlicher Konzentration enthält. Besonders bevorzugt im Rahmen dieser Erfindung sind Tetracyclin, Chloramphenicol und Erythromycin in einer Konzentration, die zwar das Wachstum sensitiver Zellen hemmt, das Wachstum der Zellen mit den entsprechenden Resistenzplasmiden aber noch erlaubt. Auf diese Weise können relativ einfach Derivate gefunden werden, die in Gegenwart der spezifisch verwendeten Selektionsmarker kein Wachstum mehr zeigen.

Zur Analyse der Restriktionsbarrieren werden die verbleibenden Isolate anschliessend mit einem geeigneten Vektor transformiert, der einerseits aus *E. coli* andererseits aus dem unmutierten Ausgangsstamm von *B. thuringiensis* und/oder *B. cereus* stammen kann. Die Transformation wird auch in diesem Fall, wie zuvor beschrieben, vorzugsweise *via* Elektroporation durchgeführt.

Auf die zuvor beschriebene Weise können somit Restriktions-defiziente Mutanten von *B. thuringiensis* bzw. *B. cereus* selektioniert werden, die bei Verwendung von Vektor-DNA aus einem heterologen Zwischenwirt, welche natürlicherweise einer Restriktion in *B. thuringiensis* und/oder *B. cereus* unterliegt, im Vergleich zu besagtem, nicht-mutierten Ausgangsstamm signifikant, vorzugsweise aber um den Faktor $\geqq 10^2$ besser transformierbar sind.

Besonders bevorzugt ist eine partiell restriktionsdefiziente Mutante des *B. thuringiensis* Stammes HD-1cryB, die, bei Verwendung von Shuttlevektoren aus einem nichtkompatiblen Zwischenwirt wie z.B. *E. coli* und-/oder *B. subtilis*, eine im Vergleich zum nicht-mutierten Ausgangsstamm um den Faktor $\geqq 10^2$ bessere Transformierbarkeit aufweist.

Ganz besonders bevorzugt im Rahmen der vorliegenden Erfindung ist die partiell restriktionsdefiziente Mutante *B. thuringiensis* var. *kurstaki* HD1cryB Res9, die durch Spontantmutation aus dem *B.t.* Stamm HD1 cryB erhältlich ist und mit Hilfe an sich bekannter Anreicherungsverfahren gewonnen werden kann sowie Mutanten und Varianten davon, die sich unmittelbar von diesem Stamm ableiten und die noch die charakteristischen Restriktions-vermindernden Eigenschaften des Ausgangsstammes aufweisen.

Die zuvor beschriebenen restriktionsdefizienten Mutanten sind aufgrund ihrer verbesserten Transformierbarkeit in hervorragender Weise geeignet für eine Verwendung als Wirtsstämme zur Klonierung und gegebenenfalls Expression von Genen oder sonstigen nützlichen DNA Sequenzen, insbesondere aber von Protoxingenen.

Im einzelnen geht man dabei vorzugsweise so vor, dass man zunächst

(a) besagte Gene oder DNA Sequenzen aus einer geeigneten Quelle isoliert oder aber diese synthetisiert;

(b) die isolierten bzw. synthetisierten Gene oder DNA Sequenzen mit Expressionssignalen, die in *Bacillus thuringiensis* und/oder *Bacillus cereus* funktionsfähig sind und die homologen oder heterologen Ursprungs sein können in Bezug auf die verwendeten Gene oder DNA Sequenzen, operabel verknüpft;

(c) die chimäre genetische Konstruktion gemäss Abschnitt (b) unter Verwendung geeigneter Vektoren, einschliesslich solcher, die natürlicherweise einer Restriktion in *B. thuringiensis* und/oder *B. cereus* unterliegen, in eine restriktionsdefiziente Mutante von *Bacillus thuringiensis* und/oder *Bacillus cereus* transformiert; und

(d) gegebenenfalls ein entsprechendes Genprodukt exprimiert und, sofern gewünscht, isoliert.

Zur weiteren Steigerung der Effizienz des erfindungsgemässen Verfahrens kann man einen zusätzlichen Verfahrensschritt zwischenschaltet, der dadurch gekennzeichnet ist, dass man die Vektor-DNA zusammen mit einer spezifischen Methylase, die in der Lage ist eine oder mehrere Basen innerhalb der Erkennungssequenz einer wirtsspezifischen Restriktionsendonuklease zu methylieren, in einem geeigneten Reaktionsansatz *in vitro* inkubiert und anschliessend die nunmehr methylierte Vektor-DNA in eine restriktionsdefiziente Mutante von *B. thuringiensis* und/oder *B. cereus* transformiert.

Besonders bevorzugt im Rahmen dieser Erfindung ist ein Verfahren zur Klonierung und gegebenenfalls Expression von Genen oder sonstigen nützlichen DNA Sequenzen, in dem man Vektoren verwendet, die aus *E. coli* oder *B. subtilis* stammen.

7

Neben Strukturgenen können dabei selbstverständlich auch beliebige andere nützliche DNA Sequenzen verwendet werden, wie z.B. nicht-kodierende DNA Sequenzen, die eine regulatorische Funktion erfüllen. Beispielhaft genannt sein an dieser Stelle eine 'anti-sense' DNA, die zwar in RNA transkribiert, nicht aber in Protein translatiert wird.

Mit Hilfe der restriktionsdefizienten Mutanten können darüberhinaus jetzt erstmals repräsentative Genbanken routinemässig in *B. thuringiensis* und/oder *Bacillus cereus* erstellt werden, wobei man vorzugsweise so vorgeht, dass man zunächst

(a) die Gesamt-DNA von *Bacillus thuringiensis* mechanisch oder vorzugsweise mit Hilfe geeigneter Restriktionsenzyme in Fragmente zerlegt;

(b) Fragmente geeigneter Grösse isoliert;

(c) besagte Fragmente in einen geeigneten Vektor, einschliesslich solchen, die natülicherweise einer Restriktion in *B. thuringiensis* und/oder *B. cereus* unterliegen, einspleisst;

(d) restriktionsdefiziente *Bacillus thuringiensis* und/oder *Bacillus cereus* Zellen mit besagtem Vektor transformiert; und

(e) aus den Transformanten mit Hilfe geeigneter Screeningverfahren diejenigen ausliest, die neue und gewünschte DNA Sequenzen enthalten.

Auch in diesem Fall lässt sich die Effizienz der Transformation durch zusätzliche Methylierung der einzuschleusenden DNA nochmals steigern.

Besonders bevorzugt im Rahmen der vorliegenden Erfindung ist dabei ein Verfahren das sich dadurch kennzeichnet, dass es sich bei besagtem *Bacillus thuringiensis* um einen Stamm handelt, der ein vergleichbares Restriktions/Modifikationssystem aufweist wie der *Bacillus thuringiensis* Stamm HD1cryB.

Einen weiteren Gegenstand dieser Erfindung bilden Verfahren zur Reduktion von Restriktionsbarrieren bei *B. thuringiensis* und/oder *B. cereus* unter Verwendung restriktions-negativer Mutanten, insbesondere aber unter Verwendung der restriktionsnegativen Mutanten *B. thuringiensis* HD1cryB Res9.

Eine weitere Steigerung der Effizienz dieses Verfahrens durch nochmalige Reduktion der Restriktionsbarrieren lässt sich dadurch erreichen, dass man eine restriktionsnegative Mutante, insbesondere aber die restriktionsnegative Mutante *B. thuringiensis* HD1cryB Res9, in Kombination mit einer spezifischen Methylase verwendet, die unter Methylierung der eingeschleusten Vektor-DNA diese vor Verdau durch *B.thuringiensis*- bzw. *B. cereus*-eigene Restriktionsenzyme schützt und somit die Effizienz des Verfahrens weiter erhöht.

Im einzelnen kann man dabei so vorgehen, dass man die Vektor-DNA zusammen mit einer spezifischen Methylase, die in der Lage ist eine oder mehrere Basen innerhalb der Erkennungssequenz einer wirtsspezifischen Restriktionsendonuklease zu methylieren, in einem geeigneten Reaktionsansatz *in vitro* inkubiert und anschliessend die methylierte Vektor-DNA in eine restriktionsdefiziente Mutante von *B. thuringiensis* und/oder *B. cereus*, insbesondere aber in Restriktions-defiziente Mutante *B. thuringiensis* HD1CryBRes9, transformiert.

Ein Beispiel einer solchen Methylase, das aber in keiner Weise als limitierend angesehen werden darf, ist das Enzym M-FnuDII, welches spezifisch das erste Cytosin innerhalb der Erkennungssequenz des Restriktionsenzyms FnuDII bzw. seiner Isoschizomeren wie z.B. BthKI [*CGCG] methyliert und daher die mit besagter Methylase behandelte Vektor-DNA vor Verdau durch dieses Enzym schützt. Auf diese Weise ist es möglich, die nach Ausschaltung der Dam-spezifischen Restriktion noch verbleibende Restaktivität, die immerhin noch für eine Reduktion der Transformationsfrequenz unmodifizierter DNA um einen Faktor 10-50 verantwortlich ist, signifikant zu reduzieren bzw ganz zu eliminieren.

Zur Illustration der eher allgemeinen Beschreibung sowie zum besseren Verständnis der vorliegenden Erfindung soll nunmehr auf spezifische Ausführungsbeispiele Bezug genommen werden, die jedoch keinen limitierenden Charakter haben, es sei denn, es wird speziell darauf hingewiesen. Das gleiche gilt auch für alle beispielhaften Aufzählungen, die in der vorangegangenen Beschreibung enthalten sind.

## NICHTLIMITIERENDE AUSFÜHRUNGSBEISPIELE

**Beispiel 1**: Isolation einer restriktions-negativen Mutante des Stammes HD1cryB

### 1.1 Für die Mutantenselektion verwendete Shuttlevektoren

Für die Isolierung einer restriktionsdefizienten Mutanten des *Bacillus thuringiensis* Stammes HD1cryB [Stahly DP *et al* (1978)] werden die folgenden *E. coli/B. thuringiensis*-Shuttlevektoren, die alle aus *E. coli* isoliert wurden, in der im folgenden aufgeführten Reihenfolge verwendet:

| Vektor | Selektion (in *B.thuringiensis*) | Herkunft/Referenz |
| --- | --- | --- |
| 1. pAM401 | Cm | Wirth *et al*, 1986 |
| 2. pHY300PLK | Tc | Toyobo Co., Osaka, 530 Japan, Bestell-Nr. PHY-001 |
| 3. pAM401 | Cm | Wirth *et al*, 1986 |
| 4. pHP13 | Em, Cm | Bacillus Genetic Stock Center, Ohio State Univ., Columbus, Ohio 43210, USA, Stamm Nr. 1P50 |

Da das Plasmid pAM401 extrem instabil ist und ohne Selektion schnell wieder verlorengeht, kann es mehr als einmal zur Transformation verwendet werden. Da auch die anderen Shuttlevektoren in *B. thuringiensis* eine gewisse Instabilität aufweisen, können später von potentiell Restriktions-negativen Mutanten relativ leicht Plasmid-freie Derivate gewonnen werden.

### 1.2 Transformation von Bacillus thuringiensis HD1cryB

Die Transformation von *Bacillus thuringiensis mit* den unter 1.1 aufgelisteten Shuttlevektoren wird mit Hilfe der Elektroporation durchgeführt.

### 1.2.1 Standard-Protokoll für die Transformation von B. thuringiensis HD1 cryB via Elektroporation

10 ml eines LB Mediums (Trypton 10 g/l, Hefeextrakt 5 g/l, NaCl 5 g/l) werden mit Sporen von *B. thuringiensis* var. kurstaki HD1cryB [Stahly DP *et al* (1978)], einer plasmidfreien Variante von *B. thuringiensis* var. kurstaki HD1, inokuliert.

Dieser Ansatz wird über Nacht bei einer Temperatur von 27°C auf einer Rundschüttelmaschine bei 50 Upm inkubiert. Danach wird die *B. thuringiensis* Kultur in 100 ml bis 400 ml LB-Medium 100fach verdünnt und bei einer Temperatur von 30°C auf einer Rundschüttelmaschine bei 250 Upm weiter kultiviert, bis eine optische Dichte ($OD_{550}$) von 0.2 erreicht ist.

Die Zellen werden mit Hilfe einer Zentrifugation geerntet und in 1/40 Volumen eines eisgekühlten PBS-Puffers (400 mM Saccharose, 1 mM $MgCl_2$, 7 mM Phosphat-Puffer pH 6.0) suspendiert. Die Zentrifugation und anschliessende Suspension der geernteten *B. thuringiensis*-Zellen in PBS-Puffer wird einmal wiederholt.

Die so vorbehandelten Zellen können dann entweder direkt elektroporiert oder aber nach Zugabe von Glycerin in die Pufferlösung [20% (w/v)], bei -20°C bis -70°C aufbewahrt und zu einem späteren Zeitpunkt verwendet werden.

400 µl Aliquots der eisgekühlten Zellen werden dann in vorgekühlte Küvetten überführt, anschliessend wird Plasmid DNA in einer geeigneten Konzentration zugegeben und der gesamte Ansatz 0.1 - 10 Minuten bei 4°C inkubiert.

Bei Verwendung von tiefgekühltem Zellmaterial wird zunächst ein geeignetes Aliquot gefrorener Zellen in Eis oder bei Raumtemperatur aufgetaut. Die weitere Behandlung erfolgt analog dem Vorgehen bei Verwendung frischen Zellmaterials.

Danach wird die Küvette in eine Elektroporationsapparatur eingebracht, wo die in der Suspension vorliegenden *B. thuringiensis*-Zellen einer Elektroporation unterzogen werden, indem sie durch einmaliges Entladen eines Kondensators mit Spannungen zwischen 0.1 kV und 2.5 kV beaufschlagt werden. Bevorzugt ist eine Spannung von 1.3 kV.

Der hier verwendete Kondensator weist eine Kapazität von 25 µF auf, die Küvetten haben einen Elektrodenabstand von 0.4 cm, was bei einer Entladung je nach Einstellung zu einer exponentiell abnehmenden Feldstärke mit anfänglichen Spitzenwerten von 0.25 kV/cm bis 6.25 kV/cm, insbesondere aber von 3.25 kV/cm führt. Die exponentielle Abklingzeit liegt in einem Bereich zwischen 10 ms und 20 ms.

Für die beschriebenen Elektroporationsversuche kann beispielsweise ein Elektroporationsgerät der Firma Bio Rad verwendet werden ('Gene Pulser Apparatus', #165-2075, Bio Rad, 1414 Harbour Way South, *Richmond*, CA 94804, USA). Selbstverständlich ist auch jedes andere geeignete Gerät in dem vorgehend beschriebenen Elektroporationsverfahren einsetzbar.

Nach einer weiteren 0.1 - 10 minütigen Inkubation bei 4°C wird die Zellsuspension mit 1.6 ml LB Medium verdünnt und 2 Stunden bei einer Temperatur von 30°C auf einer Rundschüttelmaschine bei 250 Upm inkubiert.

Anschliessend werden geeignete Verdünnungen auf L-Agar (LB Medium verfestigt mit Agar, 15 g/l) ausplattiert, der ein für die Selektion des neu erhaltenen Plasmids geeignetes Antibiotikum als Zusatz enthält. Im Falle von pHY300PLK handelt es sich dabei um das Antibiotikum Tetracyclin, das dem Medium in einer Konzentration von 20 mg/l zugegeben wird.

*Bacillus cereus* Zellen können in gleicher Weise wie *B. thuringiensis* Zellen nach obigem Protokoll transformiert werden.

### 1.2.2 Spezifische Modifikationen innerhalb der einzelnen Transformations/Selektionsrunden

1. Elektroporation: Der *Bacillus thuringiensis* Stamm HD1cryB wird gemäss Standardprotokoll [vergl. Abschnitt 1.2.1] angezüchtet und zur Erzielung grosser Transformationsfrequenzen 500fach konzentriert. [Detaillierte Angaben zur Elektroporation von *Bacillus thuringiensis* Zellen können der Europäischen Patentanmeldung EP-A 0 342 633 entnommen werden].

Nach Elektroporation der *Bacillus thuringiensis* HD1cryB Zellen mit 5 μg pAM401 Plasmid-DNA werden die Zellen 5fach in LB-Medium verdünnt und während 2 h bei 250 rpm und 30°C inkubiert. Die Transformanten werden darauf während 4 h bei 250 rpm und 30°C mit 20 μg/ml Chloramphenicol selektioniert und für die nächste Elektroporation 200fach in LB-Medium verdünnt.

2. Elektroporation: Die Zellen werden nach 2 h Inkubation bei 150 rpm und 30°C gemäss Standard-Protokoll [siehe Abschnitt 1.2.1] gewaschen und 400fach konzentriert. Nach Elektroporation mit 12 μg pHY300PLK Plasmid-DNA werden die Zellen 5fach in LB-Medium verdünnt und während 1.5 h bei 250 rpm und 30°C inkubiert. Nach weiterer 5facher Verdünnung werden die Tranformanten über Nacht bei 150 rpm und 30°C mit 20 μg/ml Tetracyclin selektioniert und für die nächste Elektroporation 200fach in LB-Medium verdünnt.

3. Elektroporation: Die Zellen werden nach 1.5 h Inkubation bei 250 rpm und 30°C gemäss Protokoll gewaschen und 100fach konzentriert. Nach Elektroporation mit 5 μg pAM401 Plasmid-DNA werden die Zellen, wie für die 2. Elektroporation beschrieben, in LB verdünnt, inkubiert, nochmal mit LB verdünnt und die Transformanten während 2.5 h bei 250 rpm und 30°C mit 10 μg Chloramphenicol selektioniert. Die auf diese Weise selektionierte Kultur wird anschliessend bei Raumtemperatur gelagert.

4. Elektroporation: Die obige Kultur wird 50fach verdünnt und gemäss Standardprotokoll über Nacht inkubiert und anschliessend als Subkultur weitergezüchtet, gewaschen und konzentriert. Nach Elektroporation mit 5 μg pHP13 werden die Zellen 5fach verdünnt und während 3.5 h bei 250 rpm und 30°C inkubiert. Die Zellen werden anschliessend erneut verdünnt und auf L-Agar mit 200 μg/ml Erythromycin ausplattiert.

Die 3. und 4. Elektroporation weisen bereits schon deutlich erhöhte Transformationsfrequenzen auf, was einen ersten Hinweis auf eine Anreicherung Restriktions-definzierter Mutanten darstellt.

### 1.3 Herstellung Plasmid-freier Derivate

Zur weiteren Beurteilung potentiell restriktionsdefizienter Mutanten werden zunächst Plasmid-freie Derivate hergestellt. Dazu werden 10 Einzelkolonien von den obigen Selektionsplatten gepickt [nach der 4. Elektroporation, siehe Abschnitt 1.2.2] und in 10 ml LB-Medium über Nacht bei 50 rpm und 27°C ohne Selektion inkubiert.

Die Kulturen werden anschliessend verdünnt und auf L-Agar ohne Selektivmarker ausplattiert. Geeignete Verdünnungen dieser Kulturen werden dann auf L-Agar-Platten repliziert, die jeweils einen der folgenden Selektionsmarker enthalten: Tetracyclin [20 μg/ml], Chloramphenicol [ 10 μg/ml] oder Erythromycin [200 μg/ml].

Von 6 der ursprünglichen 10 Kolonien können auf diese Weise relativ einfach Derivate gefunden werden, die in Gegenwart aller drei Selektionsmarker kein Wachstum mehr zeigen. In keinem dieser Derivate kann noch eines der zuvor eingeschleusten Plasmide nachgewiesen werden.

### 1.4 Transformation mit dem Shuttlevektor pHY300PLK

Zur Analyse der Restriktionsbarrieren werden die verbleibenden 6 Isolate mit pHY300PLK transformiert, das einerseits aus *E. coli* andererseits aus *B. thuringiensis* HD1cryB stammt. Die Transformation wird *via* Elektroporation durchgeführt [vergl. Beispiel 1.2.1].

Die Ergebnisse der Tab. 3 zeigen, dass 4 der 6 getesteten Isolate eine gegenüber dem Elternstamm HD-

1cryB deutlich erniedrigte Restriktionsbarriere aufweisen. Von diesen mit Res 5, 7, 8 und 9 bezeichneten Mutanten weisen Res 5, 7 und 9 eine ca. 50-100x kleinere Restriktionsbarriere auf als HD1cryB. Res 5 und 7 sind aber absolut gesehen 10x schlechter transformierbar als HD1cryB. Res 8 zeigt zwar eine extrem niedrige Restriktionsbarriere, ist aber absolut gesehen ca. 500x schlechter transformierbar als HD1cryB, was den Vorteil der erniedrigten Restriktionsbarriere wieder aufhebt.

Lediglich die restriktions-negative Mutante Res 9 kombiniert die Vorteile einer hohen Transformationseffizienz mit denjenigen erniedrigter Restriktionsbarrieren.

Die Vorteile der Mutanten Res9 werden noch deutlicher bei Transformationen mit rekombinanten Plasmiden. Aus Tabelle 4 wird deutlich, dass aus *E. coli* isolierte Shuttlevektoren, die ein Protoxingen enthalten [pXl204, pXl93], nur mit sehr geringer Frequenz in *B. thuringiensis* HD1cryB transformiert werden können, während die Mutante Res 9 unter Verwendung der gleichen Plasmide eine effiziente Transformation ermöglicht.

1.5 Taxonomische Charakterisierung der verwendeten Bacillusthuringiensis Stämme

1.5.1 Stamm HD1cryB

Identifizierung von Stamm DSM 4574

Eigenschaften des Stammes

Stäbchen
    Breite /µm                                       1,0-1,2
    Länge /µm                                          3,0-5,0

Beweglichkeit                                       +

Sporen                                          +
    ellipsoid                                       +
    rund                                         -
    Sporangium angeschwollen               -

Gram Reaktion                                   +

Catalase                                       +

Anaerobes Wachstum                          +

VP Reaktion                                    +

pH in VP-Medium                               4,9

Maximale Temperatur
    Wachstum positiv bei °C                 45
    Wachstum negativ bei °C               50

Wachstum in
    Medium pH 5.7                         +
    NaCl   5 %                             +

7 %      -

10 %      -

Säure aus

  Glucose      +

  L-Arabinose      -

  Xylose      -

  Mannit      -

Gas aus Glucose      -

Lecithinase      -

Hydrolyse von

  Stärke      +

  Gelatine      +

  Casein      +

Verwertung von

  Citrat      +

  Propionat      -

Abbau von Tyrosin      -

NO2 aus NO3      +

Indol      -

Phenylalanindesaminase      -

Arginindihydrolase      +

ungewöhnliche Eigenschaft:

keine Lecithinase-Aktivität

1.5.2 Stamm HD1cryB Res9

Identifizierung von Stamm DSM 5854

Eigenschaften des Stammes

| | |
|---|---|
| Stäbchen | |
| Breite /μm | 1,0-1,2 |
| Länge /μm | 3,0-5,0 |
| Beweglichkeit | + |
| Sporen | + |
| ellipsoid | + |
| rund | - |
| Sporangium angeschwollen | - |
| Gram Reaktion | + |
| Catalase | + |
| Anaerobes Wachstum | + |
| VP Reaktion | + |
| pH in VP-Medium | 4,7 |
| Maximale Temperatur | |
| Wachstum positiv bei °C | 45 |
| Wachstum negativ bei °C | 50 |
| Wachstum in | |
| Medium pH 5.7 | + |
| NaCl   5 % | + |
| 7 % | - |
| 10 % | - |

14

Säure aus

   Glucose                                         +

   L-Arabinose                                  –

   Xylose                                        –

   Mannit                                        –

Gas aus Glucose                            –

Lecithinase                                 –

Hydrolyse von

   Stärke                                        +

   Gelatine                                    +

   Casein                                        +

Verwertung von

   Citrat                                        +

   Propionat                                  –

Abbau von Tyrosin                      –

NO2 aus NO3                            +

Indol                                        –

Phenylalanindesaminase                  –

Arginindihydrolase                     +

ungewöhnliche Eigenschaft:

keine Lecithinase-Aktivität

**Beispiel 2:** Charakterisierung der Restriktionsbarrieren der Stämme HD1cryB und Res 9

2.1 Restriktionsenzym BthK1

In Rohextrakten des Stammes HD1cryB ist es unmöglich Sequenz-spezfische Restriktionsenzyme nachzuweisen, da die Test-DNA von unspezifischen Nukleasen total degradiert wird. Diese interferierenden Nukleasen können auf einfache Weise durch eine Dextran-Polyethylenglycoll Phasenverteilung grösstenteils abgetrennt werden [Schleif (1980)]. Je nach den Eigenschaften der vorhandenen Nukleasen und Restriktion-sendonukleasen können Ionenstärken gefunden werden, bei denen die verschiedenen Enzyme verschiedene Phasen vorziehen. Im Falle von HD1cryB können die unspezifischen Nukleasen bei allen Ionenstärken entfernt werden. Bei NaCl-Konzentrationen kleiner als 10 mM oder grösser als 250 mM kann in der wässrigen Phase eine sequenzspezifische Nuklease nachgewiesen werden.

Eine weitere Reinigung und Auftrennung der in Stamm HD1cryB vorhandenen Restriktionsenzyme wurde durch Affinitätschromatographie an einer Heparin-Säule [Bickle (1977)] wie folgt vorgenommen. Aus 5 Litern Kultur werden die Zellen durch Zentrifugation geerntet, in einem Zellaufschlussgerät [z.B. eine French Pressure Cell] aufgeschlossen und die Zelltrümmer durch Zentrifugation abgetrennt. Nukleinsäuren werden durch Fällung mit Polyethylenimin (Pirrotta, 1980) abgetrennt und die Proteine mit $(NH_4)_2SO_4$ bei 70 % Sättigung gefällt und in einem kleinen Volumen Puffer aufgenommen. Die Proteine werden anschliessend auf eine Heparin-Säule geladen und mit einem NaCl-Gradienten [0 M-1 M] eluiert. Aliquots der eluierten Fraktionen werden anschliessend auf Anwesenheit von Restriktionsenzymen getestet [Bickle (1977)]. Als Substrat dient aus *E. coli* isoliertes pHY300PLK, ein Plasmid das bekanntermassen der Restriktion unterliegt. Trotz unvollständiger Trennung von den unspezifischen Nukleasen konnte eine Sequenz-spezifische Restriktionsaktivität gezeigt werden, die ein definiertes Muster von DNA-Fragmenten erzeugt. Die Restriktionsaktivität eluiert bei ca. 0.6 M -0.8 M.

Das angereicherte Enzym schneidet das Plasmid pHY300PLK, wenn es zuvor aus *E. coli* oder *B. subtilis* isoliert wurde, nicht aber, wenn es aus HD1cryB isoliert wurde. Charakteristischerweise ist also auch in diesem Fall der Produzent des Restriktionsenzyms vor Verdau seiner eigenen DNA geschützt, wahrscheinlich durch Sequenz-spezifische Methylierung.

Die zuvor dokumentierten Eigenschaften weisen somit alle auf ein Restriktionsenzym der Klasse II hin. In Uebereinstimmung mit der akzeptierten Nomenklatur [Szybalski *et al* (1988)] erhält das aus *B. thuringiensis* HD1cryB isolierte Enzym die Bezeichnung BthK1.

Auch dieses Restriktionsenzym BthK1 bildet einen Gegenstand der vorliegenden Erfindung.

2.1.1 Spezifische Erkennungssequenz von BthK1

Die spezifische Erkennungs- und Schnitt-Sequenz des aus HD1cryB isolierten Restriktionsenzyms kann folgendermassen bestimmt werden.

Die als Substrat verwendete DNA wird im allgemeinen in viele kleine Fragmente zerschnitten, was vermuten lässt, dass es sich um ein Enzym handelt, dessen Erkennungs-Sequenz nur aus vier Nukleotiden besteht. Zudem wird DNA mit mittlerem bis hohem GC-Gehalt häufig, DNA mit kleinem GC-Gehalt eher weniger häufig geschnitten, was wiederum den Schlüss zulässt, dass die Erkennungs-Sequenz reich an GC ist. Bei einem Enzym das eine Vierer-Sequenz erkennt bedeutet das, dass die Sequenz ausschliesslich aus GC besteht. Unter Benützung von aus *B. subtilis* isolierter DNA der Plasmide pC194 [Horinuchi und Weisblum (1982)] und pBC16 [Bernhard *et al* (1978)] als Substrat und durch Vergleich mit kommerziell erhältlichen Restriktionsenzymen konnte eindeutig gezeigt werden, dass das Restriktionsenzym BthK1 ein Isoschizomer der kommerziell erhältlichen Enzyme Tha1 [GIBCO BRL, Gaithersburg, Maryland 20877, USA], Acc2 [Stratagene, La Jolla, CA 92037, USA], BstU1 [New England Biolabs, Beverly, MA 01915-5510, USA] und Mvn1 [Boehringer Mannheim, D-6800 Mannheim, BRD] ist, d.h. dass es die Sequenz CGCG erkennt und schneidet, falls diese nicht spezifisch methyliert ist. Die für das Restriktionsenzym BthK1 postulierte spezifische Methylase M.BthK1 schützt nicht nur vor Verdau durch BthK1 sondern auch vor Verdau durch Tha1 oder Acc2. Da die Methylierung dieser beiden Enzyme bekannt ist, kann geschlossen werden, dass M.BthK1 zumindest das erste, möglicherweise aber auch beide C in der Sequenz CGCG methyliert.

Die Restriktionsenzyme in Stamm Res 9 wird auf die gleiche Weise analysiert wie oben für HD1cryB beschrieben wurde. Das Elutionsprofil einer Heparin-Sepharose Affinitätssäule ist bei Res 9 gleich wie bei HD-1cryB, d.h. BthK1 wird immer noch produziert und ist darum nicht der Grund für die verringerte Restriktionsbarriere bei Res 9. Die dafür verantwortliche Mutation bleibt also immer noch ungeklärt.

2.2 Methylierungs-spezifische Restriktion

Spezifische Methylierung von Adenin oder Cytosin erfolgt in *E. coli* K durch die <u>Dam</u>- oder <u>Dcm</u>-Methylase. *E. coli* B- Stämme sind natürlicherweise dcm⁻. Restriktionsenzyme die die gleiche Sequenz erkennen wie die <u>Dam</u>- oder die <u>Dcm</u>-Methylase können je nach Typ durch diese Methylierung gehemmt werden. Diese Restriktionsenzyme können also diagnostisch eingesetzt werden zur Abklärung des Methylierungs-Status einer bestimmten DNA. Mit dieser Methode kann man zeigen, dass sowohl *B. subtilis* als auch *B. thuringiensis* weder <u>Dam</u>- noch <u>Dcm</u>-Methylierung aufweisen.

Hinweise auf die Natur der Mutation im Stamm Res 9 können durch Verwendung von DNA des Shuttlevektors pHY300PLK, der aus Stämmen mit unterschiedlichem <u>Dam/Dcm</u>-Phänotyp isoliert wurde, gewonnen werden. Tab. 5 zeigt die Transformationsfrequenzen dieser DNA in die Stämme HD1cryB und Res 9. Der <u>Dam/Dcm</u>-Phänotyp der DNA wurde in jedem Fall sorgfältig durch Verdauen mit den entsprechenden Methylierungs-empfindlichen Restriktionsenzymen bestätigt.

Die Resultate aus Tab. 5 können wie folgt zusaminengefasst und interpretiert werden.

<u>Dam</u>-Methylierung der DNA ist verantwortlich für den Hauptteil der in HD1cryB beobachteten Restriktionsbarrieren. Res 9 hingegen wird durch den <u>Dam</u>-Phänotyp nicht beeinflusst und nimmt sowohl <u>Dam</u>-methylierte als auch unmethylierte DNA gleich gut auf.

Die <u>Dcm</u>-Methylierung hat somit offensichtlich keinen Einfluss auf die Transformierbarkeit von *B. thuringiensis*HD1cryB. Da keine isogenen Stämme zur Verfügung standen, können die kleineren aber doch reproduzierbaren Unterschiede zwischen den einzelnen Wirtsstämmen nicht durch einen einzelnen Phänotyp erklärt werden. Es bleibt aber eine interessante Beobachtung, dass DNA aus verschiedenen Wirtsstämmen unterschiedlich gut transformierbar ist.

Durch *in vitro* Methylierung mit kommerziell erhältlicher <u>Dam</u>-Methylase kann bestätigt werden, dass die <u>Dam</u>-Methylierung tatsächlich der Grund ist für einen Teil der Restriktionsbarrieren in HD1cryB (Tab. 6). *In vitro* Methylierung mit drei anderen Methylasen zeigt auch, dass nicht eine allgemeine Methylierung sondern spezifisch die <u>Dam</u>-Methylierung die Restriktionsbarriere verursacht.

**Beispiel 3:** Weitere Reduktion der Restriktionsbarrieren durch spezifische <u>Methylierung</u>

Von den zwei im *Bacillus thuringiensis* Stamm HD1cryB vorliegenden Restriktionssystemen kann die Hauptaktivität, die auf einer <u>Dam</u>-spezifischen Restriktion basiert, durch eine Mutation im Derivat Res9 inaktiviert werden. Die noch verbleibende Restaktivität, die immerhin noch für eine Reduktion der Transformationsfrequenz unmodifizierter DNA um eine Faktor 10-50 verantwortlich ist und auf dem Restriktionsenzym BthKI basiert, kann durch spezifische Methylierung mit der Methylase M-FnuDII signifikant reduziert werden.

3.1 Spezifische Methylierung des Shuttlevektors pHY300PLK mit M-FnuDII <u>Methylase</u>

Die von New England Biolabs vertriebene Methylase M-FnuII [Nr. 224S, New England Biolabs, 32 Tozer Road, Beverly, MA 01915-5599, USA] methyliert spezifisch die Sequenz *CGCG und schützt damit vor Verdau mit FnuDII und seinen Isoschizomeren wie z.B. BthKI.

DNA des Shuttlevektors pHY300PLK wird aus *Bacillus subtilis* oder E. coli isoliert und in folgendem Ansatz methyliert:

| | |
|---|---|
| DNA pHY300PLK | 0.3 µg |
| Tris-HCl [pH 7.5] | 50 mM |
| EDTA | 10 mM |
| β-Mercaptoethanol | 5 mM |
| S-Adenosinmethionin | 0.08mM |
| M-FnuDII | 2 UN* |

*UN Methylierungseinheiten. Eine Einheit entspricht derjenigen Menge Enzym, die benötigt wird um ein µg Lambda DNA [in einer Stunde/37°C, in 10 µl Reaktionsvolumen) vollständig vor Verdau durch FnuDII zu schützen.

EP 0 471 647 A2

Die Methylierung wird während einer Stunde bei einer Temperatur von 37°C durchgeführt Die Methylierungsreaktion wird abgebrochen durch Inaktivierung der Methylase. Dazu wird der gesamte Ansatz ein weiteresmal inkubiert, und zwar für 20 min bei einer Temperatur von 65°C. Die DNA wird anschliessend durch Zugabe von Ethanol gefällt und 10 µl TE-Puffer resuspendiert. Jeder Ansatz wird in dreifacher Ausfertigung durchgeführt, wobei jeweils 1 Aliquot für die sich anschliessende Transformation, ein weiteres für die Überprüfung der Plasmid-DNA auf Unversehreit verwendet wird. Letztere erfolgt mittels Agarose-Gelelektrophorese.

Die Wirksamkeit der Methylierung wird durch Verdau der Plasmid DNA mit dem FnuDII Isoschizomer ThaI getestet. Die Ergebnisse zeigen, dass die DNA vor Verdau mit ThaI geschützt ist, d.h. dass sie vollständig methyliert ist, während die nicht methylierten Kontrollen durch das Enzym verdaut werden.

3.2 Kombination von Mutation und Methylierung

Die zuvor in Abschnitt 3.1 methylierte pHY300PLK Plasmid-DNA wird gemäss dem in Beispiel 1.2.1 beschriebenen Protokol in die restriktionsdefiziente *B. thuringiensis* Mutante Res9 transformiert. Die Ergebnisse sind in Tabelle 7 wiedergegeben. Die Resultate zeigen, dass eine Kombination von Methylierung der Plasmid DNA durch M-FnuDII und Verwendung von Res9 als Wirtsstamm eine vollständige Überwindung der im *B. thuringiensis* Stamm HD1cryB vorgefundenen Restriktionsbarrieren erlaubt.

**MEDIEN UND PUFFER**

| LB-Medium | [g/l] |
|---|---|
| Trypton | 10 |
| Hefeextrakt | 5 |
| NaCl | 5 |

| L-Agar | [g/l] |
|---|---|
| LB-Medium verfestigt mit | |
| AGAR | 15 |

| Antibiotic Medium Nr. 3 (Difco Laboratories) | [g/l] |
|---|---|
| Rinder-Fleischextrakt | 1.5 |
| Hefeextrakt | 1.5 |
| Pepton | 5 |
| Glucose | 1 |
| NaCl | 3.5 |

| | |
|---|---|
| $K_2HPO_4$ | 3.68 |
| $KH_2PO_4$ | 1.32 |

| SCGY-Medium | [g/l] |
|---|---|
| Casaminosäuren | 1 |
| Hefeextrakt | 0.1 |
| Glucose | 5 |
| $K_2HPO_4$ | 14 |
| $KH_2PO_4$ | 6 |
| $Na_3$-Citrat | 1 |
| $(NH_4)_2SO_4$ | 2 |
| $MgSO_4 \cdot 7\ H_2O$ | 0.2 |

| GYS-Medium (Yousten & Rogoff, 1969) | [g/l] |
|---|---|
| Glucose | 1 |
| Hefeextrakt | 2 |
| $(NH_4)_2SO_4$ | 2 |
| $K_2HPO_4$ | 0.5 |
| $MgSO_4 \cdot 7\ H_2O$ | 0.2 |
| $CaCl_2 \cdot 2\ H_2O$ | 0.08 |
| $MnSO_4 \cdot H_2O$ | 0.05 |

pH vor Autoklavieren auf 7.3 einstellen.

| PBS-Puffer | [mM] |
|---|---|
| Saccharose | 400 |
| $MgCl_2$ | 1 |
| Phosphat-Puffer, pH 6.0 | 7 |

| TBST-Puffer | [mM] |
|---|---|
| Tween 20* | 0.05% (w/v) |
| Tris/HCl* (pH 8.0) | 10 |
| NaCl | 150 |

Puffer High

| [Maniatis et al (1982); Seite 104] | [mM] |
|---|---|
| NaCl | 100 |

| | |
|---|---|
| Tris/HCl* (pH 7.5) | 50 |
| MgCl$_2$ | 10 |
| Dithiothreitol | 1 |

| Nick-Translationspuffer (10x) | [mM] |
|---|---|
| Tris/HCl* (pH 7.2) | 500 |
| MgSO$_4$ | 100 |
| Dithiothreitol | 1 |
| Rinderserumalbumin (BSA Pentax Fraktion V) | 500 µg/ml |

| TE-Puffer | [mM] |
|---|---|
| Tris/HCl (pH 8.0) | 10 |
| EDTA | 1 |

| | |
|---|---|
| *Tween 20 | Polyethoxysorbitanlaurat |
| *Tris/HCl | $\alpha,\alpha,\alpha$-Tris(hydroxymethyl)methylaminohydrochlorid |

## HINTERLEGUNG

Die im folgenden aufgelisteten Mikroorganismen, die im Rahmen der vorliegenden Erfindung verwendet werden, wurden bei der als Internationale Hinterlegungsstelle anerkannten 'Deutschen Sammlung von Mikroorganismen' in Braunschweig, BRD, gemäss den Anforderungen des Budapester Vertrages für die Internationale Anerkennung der Hinterlegung von Mikroorganismen zum Zwecke der Patentierung, hinterlegt.

| Mikroorganismus | Hinterlegungs-datum | Hinterlegungs-Nummer | Datum der Lebensfähikgeits bescheinigung |
|---|---|---|---|
| *Bacillus thuringiensis* var. *kurstaki* HD1 | 4. März 1986 | DSM 3667 | 5. März 1986 |
| *Bacillus thuringiensis* var. *kurstaki* HD1 cryB | 4. Mai 1988 | DSM 4574 | 4. Mai 1988 |
| *Bacillus thuringiensis* var. *kurstaki* HD1 cryB Res9 | 21. März 1990 | DSM 5854 | 21. März 1990 |
| *Bacillus thuringiensis* var. *kurstaki* HD1cryB transf. mit **pK61** | 4. Mai 1988 | DSM 4572 | 4. Mai 1988 |
| *Bacillus thuringiensis* var. *kurstaki* HD1cryB tranf. mit **pK93** | 4. Mai 1988 | DSM 4571 | 4. Mai 1988 |

Da sich ein *B. thuringiensis* mit Hilfe der gewählten Kriterien nur anhand seiner parasporalen Kristalle von einem *B. cereus* unterscheiden lässt, können sowohl der Stamm HD 1cryB (DSM Nr. 4574) als auch der Restriktionsdefiziente Stamm Res 9 (DSM Nr. 5854) als *B. cereus* klassifiziert werden.

Diese Klassifizierung ist möglich, da der Stamm HD1cryB ein Plasmid-freies und damit Kristall-freies Derivat von HD1 ist (Stahly *et al.*, 1978), was automatisch eine Klassifizierung als *B. cereus* zur Folge hat. Auf Grund der bekannten Unterschiede der drei charakterisierten Stämme kann demzufolge auch keine taxonomische Identität erwartet werden. Da aber die in Abschnit 1.5 gezeigten Eigenschaften der drei Stämme auch nicht im Widerspruch zu ihrer belegten verwandtschaftlichen Herkunft steht, werden die Stämme HD1cryB und Res 9 im Rahmen der vorliegenden Erfindung auch weiterhin als *B. thuringiensis* bezeichnet.

## LITERATUR

Asisbekjan, R. R. et al., Dokl. Akad. Nauk SSSR 274, 742-744, 1984

Bailey, C. R. und Winstanley, D. J., J. Gen. Microbiol. 132, 2945-2947, 1986

Bernhard K et al., J. Bacteriol. 133, 897-903, 1978

Bickle, T. A. et al., Nucl. Acids Res. 4, 2561-2572, 1977

Bone, E. J. und Ellar, D. J., FEMS Microbiol. Letters 58, 171-178, 1989

Bron., S. et al., Mol. Gen. Genet. 211, 186-189, 1988

Engel, P., Appl. Environm. Microbiol. 53, 1-3, 1987

Haima, P. et al., Mol. Gen. Gemet. 209, 335-342, 1987

Horinuchi und Weisblum J. Bacteriol. 150, 815-825, 1982

Hoshino, T. et al., Agric. Biol. Chem. 44, 621-623, 1980

Kessler, C. und Höltke, H.-J., Gene 47, 1-153, 1986

Kretz, P. L. et al., Nucl. Acids Research 17, 5409, 1989

Lereclus, D. et al., FEMS Microbiol. Letters 60, 211-218, 1989

Macaluso, A., Abstr. Annu. Meet. Am. Soc. Microbiol., 309, 1989

MacNeil, D.J., J. Bacteriol. 170, 5607-5612, 1988

MacNeil, D.J., Europ. Patent Appl., Case Nr. 8920 1140.4, 1989

Matsushima, P. et al., Mol. Gen. Genet. 206, 393-400, 1987

Miller, J. F. et al., Proc. Natl. Akad. Sci. USA 85, 856-860, 1988

Orzech, K. et al., J. Gen. Microbiol. 130, 203-208, 1984

Pirrotta, V. und Bickle, T. A., in: Meth. Enzymol. 65, 89-95, 1980

Raleigh, E. A. und Wilson, G., Proc. Natl. Acad. Sci. USA 83, 9070-9074, 1986

Schleif, R., in: Meth. Enzymol. 65, 19-23, 1980

Schurter, W. et al., Molec. Genet. 218, 177-181, 1989

Sladek, T. L. et al., J. Bacteriol. 165, 219-225, 1986

Stahly DP et al, Biochem. Biophys. Res. Comm., 84, 581-588, 1978

Szybalski, W. et al., Gene 74, 279-280, 1988

Uozumi, T. et al., Mol. Gen Genet. 152, 65-69, 1977

Vehmaanperä, J., FEMS, Microbiol. Letters 49, 101-105, 1988

Wirth R et al, J. Bacteriol. 165, 831-836, 1986

## TABELLEN

**Tabelle 1.** Restriktionsbarrieren bei Transformation von *Bacillus thuringiensis* HD1cryB mit Shuttlevektor pHY300PLK

| pHY300PLK isoliert aus: | Transformationsfrequenzen* im Stamm HD1cryB | |
| --- | --- | --- |
| | absolut | relativ |
| *B.thuringiensis* HD1cryB | $1.2 \times 10^7$ | 1 |
| *E. coli* HB101** | $1.8 \times 10^3$ | $1.5 \times 10^{-4}$ |
| *B. subtilis* ISW1214*** | $7.6 \times 10^5$ | $6.3 \times 10^{-2}$ |

\* für den relativen Wert wird die Frequenz des aus dem Stamm HD1cryB isolierten Plasmides als 1 gesetzt.

\*\* [American Type Culture Collection (ATCC), Rockville, Maryland, USA, Stamm Nr. 33694]

\*\*\* [Toyobo Co. LTD, Osaka, 530 Japan, Bestell-Nr. PHY-001]

**Tabelle 2.** Shuttlevektoren, die zur Anreicherung von Restriktions-defizienten Mutanten von HD1cryB verwendet wurden.

| Vektor | Selektion (in HD1cryB) | gram$^+$ Replikon |
| --- | --- | --- |
| 1. pAM401 | Cm | pIP501 |
| 2. pHY300PLK | Tc | pAMα1 |
| 3. pAM401 | Cm | pIP501 |
| 4. pHP13 | Em, Cm | pTA1060 |

**Tabelle 3.** Transformations-Verhalten von **potentiellen Restriktions-defizienten Derivaten** von B. thuringiensis DH1cryB

| transformierter Stamm | Transformationsfrequenzen* von pHY300PLK isoliert aus: | | | |
| | B. thuring. HD1cryB | | E. coli BZ234 | |
| | absolut | relativ | absolut | relativ |
|---|---|---|---|---|
| **HD1cryB** | **$9.5x10^6$** | **1** | **$9.0x10^3$** | **$9.5x10^{-4}$** |
| Res 5 | $1.3x10^6$ | 1 | $1.0x10^5$ | $7.7x10^{-2}$ |
| Res 7 | $1.5x10^6$ | 1 | $1.1x10^5$ | $7.4x10^{-2}$ |
| Res 8 | $6.0x10^4$ | 1 | $2.0x10^4$ | $3.3x10^{-1}$ |
| **Res 9** | **$1.1x10^7$** | **1** | **$7.0x10^5$** | **$6.4x10^{-2}$** |

* für die Berechnung des relativen Wertes wird die Frequenz des aus dem Stamm HD1cryB isolierten Plasmides als 1 gesetzt

**Tabelle 4.** Charakterisierung der Restriktionsbarrieren der Stämme HD1cryB und Res9.
I: Transformierbarkeit von verschiedenen, aus *E.coli* isolierten, rekombinanten Plasmiden.

| Plasmid | isoliert aus | Transformationsfrequenzen in die Stämme | |
| | | HD1cryB | Res9 |
|---|---|---|---|
| pXI204 * | *B.t.* HD1cryB | $1.3 x 10^6$ | $1.5 x 10^6$ |
| pXI204 | *E.c.* HB101 | $< 8 x 10^1$ | $6.4 x 10^4$ |
| pXI93 ** | *B.t.* HD1cryB | $3.0 x 10^5$ | $8.3 x 10^5$ |
| pXI93 | *E.c.* HB101 | 4 | $1.3 x 10^4$ |

* beschrieben in EP-A 0 342 633 ;

** beschrieben in Schurter *et al* (1989) sowie EP-A 0 342 633; die in der von DSM ausgestellten Hinterlegungsbescheinigung eingetragene interne Bezeichnung pK wurde zwischenzeitlich gemäs der gültigen internationalen Klassifikation in pXI geändert.

**Tabelle 5.** Charakterisierung der Restriktionsbarrieren der Stämme **HD1cryB** und **Res 9**. II: Einfluss des Wirtsstammes, speziell dessen <u>Dam</u>/<u>Dcm</u>-Phänotyps, auf die Transformierbarkeit des Shuttlevektors pHY300PLK.

| | Phänotyp | | relative* Transformationsfrequenz in B. thuringiensis, Stamm: | |
| --- | --- | --- | --- | --- |
| Plasmid isoliert aus | Dam | Dcm | HD1cryB | Res 9 |
| B. th. HD1cryB | - | - | 1 | 1 |
| B. th. Res 9 | - | - | 1 | 1 |
| B. subtilis ISW1214 | - | - | $9.1 \times 10^{-2}$ | $8.9 \times 10^{-2}$ |
| E. coli BZ103 | - | +/- | $1.3 \times 10^{-2}$ | $0.7 \times 10^{-2}$ |
| E. coli 3225 | - | - | $0.8 \times 10^{-2}$ | $0.4 \times 10^{-2}$ |
| E. coli BL21 | + | - | $2.4 \times 10^{-4}$ | $0.7 \times 10^{-2}$ |
| E. coli HB101 | + | + | $8.2 \times 10^{-4}$ | $2.1 \times 10^{-2}$ |
| E. coli W3110 | + | + | $1.8 \times 10^{-4}$ | $0.9 \times 10^{-2}$ |

\* pHY300PLK isoliert aus HD1cryB oder Res 9 transformiert mit gleicher Frequenz in Stamm DH1cryB und wird als 1 gesetzt. Die Transformationsfrequenzen der anderen Isolate sind in Relation zu diesem Wert angegeben. Das Gleiche gilt auch für Transformationen in Stamm Res 9.

**Tabelle 6.** Sequenz-spezifische in vitro Methylierung von aus dem Stamm HD1cryB isolierter pHY300PLK-DNA: Einfluss auf das Transformationsverhalten von HD1cryB und Res 9.

| | | relative[+] Transformationsfrequenzen in Stamm: | |
| --- | --- | --- | --- |
| Methylase | methylierte Sequenz | HD1cryB | Res 9 |
| - - - | - - - | 1 | 1 |
| M.Hha I | GC* GC | 0.9 | 0.8 |
| M.Hpa II | CC* GG | 0.4 | 0.6 |
| M.Pst I | CTGCA$ G | 0.7 | 0.7 |
| Dam | GA$ TC | $3.7 \times 10^{-3}$ | 0.8 |

[+] die Transformationsfrequenzen in Stamm HD1cryB und Res 9 wurden je als 1 gesetzt

\* 5-Methylcytosin

$ $N^6$-Methyladenin

**Tabelle 7.** Transformation von *B.thuringiensis* HD1cryBRes9: Effekt der Methylierung von pHY300PLK-DNA durch die Methylase M-FnuDII

| Herkunft des Plasmids | Behandlung der DNA | relative Transformationsfrequenz |
|---|---|---|
| *B.t.* HD1cryB | - - - - - | 1 |
| *B.s.* ISW1214 | - - - - - | 0.06 |
| *B.s.* ISW1214 | Kontrolle | 0.02 |
| *B.s.* ISW1214 | M-FnuDII | 0.58 |
| *E.coli* TG1* | - - - - - - | 0.0031 |
| *E.coli* TG1* | Kontrolle | 0.0029 |
| *E.coli* TG1* | M-FnuDII | 0.32 |

\* Teil des *in vitro* Mutagenese-Kits Nr. RPN1523 der Firma AMERSHAM, Buckinghamshire HP7 9NA, England

**Patentansprüche**

1. Mutante von *B. thuringiensis* und/oder *B. cereus*, die eine partielle Defiziens innerhalb der inhärent im unmutierten Ausgangsstamm vorliegenden Restriktionsbärrieren aufweist und daher bei Verwendung von Vektor-DNA aus einem heterologen Zwischenwirt, die natürlicherweise einer Restriktion in *B. thuringiensis* und/oder *B. cereus* unterliegt, im Vergleich zu besagtem, nicht-mutierten Ausgangsstamm signifikant besser transformierbar ist.

2. Partiell restriktionsdefiziente Mutante gemäss Anspruch 1, dadurch gekennzeichnet, dass es sich bei besagtem heterologen Zwischenwirt um einen nicht-kompatiblen Zwischenwirt handelt.

3. Partiell restriktionsdefiziente Mutante gemäss Anspruch 2, dadurch gekennzeichnet, dass es sich bei besagtem nicht-kompatiblen Zwischenwirt um *E. coli* oder *Bacillus subtilis* handelt.

4. Partiell restriktionsdefiziente Mutante gemäss Anspruch 1, dadurch gekennzeichnet, dass es sich bei besagtem unmutierten Ausgangsstamm um den *B. thuringiensis* Stamm HD1cryB handelt.

5. Partiell restriktionsdefiziente Mutante gemäss Anspruch 1, dadurch gekennzeichnet, dass besagte Mutante eine im Vergleich zum nicht-mutierten Ausgangsstamm um den Faktor $\geqq 10^2$ bessere Transformierbarkeit aufweist.

6. Partiell restriktionsdefiziente Mutante gemäss Anspruch 1, welche die folgenden, kennzeichnenden, taxonomischen Charakteristika aufweist:

Stäbchen

Breite /μm          1,0-1,2

Länge /μm          3,0-5,0

Beweglichkeit          +

| | |
|---|---|
| Sporen | + |
| ellipsoid | + |
| rund | - |
| Sporangium angeschwollen | - |
| Gram Reaktion | + |
| Catalase | + |
| Anaerobes Wachstum | + |
| VP Reaktion | + |
| pH in VP-Medium | 4,7 |
| Maximale Temperatur | |
| Wachstum positiv bei °C | 45 |
| Wachstum negativ bei °C | 50 |
| Wachstum in | |
| Medium pH 5.7 | + |
| NaCl    5 % | + |
| 7 % | - |
| 10 % | - |
| Säure aus | |
| Glucose | + |
| L-Arabinose | - |
| Xylose | - |
| Mannit | - |
| Gas aus Glucose | - |
| Lecithinase | - |
| Hydrolyse von | |

| | |
|---|---|
| Stärke | + |
| Gelatine | + |
| Casein | + |

Verwertung von

| | |
|---|---|
| Citrat | + |
| Propionat | - |

| | |
|---|---|
| Abbau von Tyrosin | - |
| NO2 aus NO3 | + |
| Indol | - |
| Phenylalanindesaminase | - |
| Arginindihydrolase | + |

ungewöhnliche Eigenschaft:
keine Lecithinase-Aktivität

sowie Mutanten und Varianten davon, die sich unmittelbar oder mittelbar von diesem Stamm ableiten und die noch die charakteristischen restriktionsvermindernden Eigenschaften des Ausgangsstammes aufweisen.

7. Die restriktionsdefiziente Mutante *B. thuringiensis* var. *kurstaki* HD1cryB Res9, welche die kennzeichnenden Charakteristika von DSM 5854 aufweist sowie Mutanten und Varianten davon, die sich unmittelbar oder mittelbar von diesem Stamm ableiten und die noch die charakteristischen Restriktions-vermindernden Eigenschaften des Ausgangsstammes besitzen.

8. Verfahren zur Herstellung einer restriktionsdefizienten Mutanten von *B. thuringiensis* und/oder *B. cereus*, dadruch gekennzeichnet dass man Spontanmutanten mit verminderter Restriktionsbarriere anreichert, indem man mehrere Transformations- und Selektionsrunden hintereinanderschaltet, wobei man für die Transformation vorzugsweise Shuttlevektoren verwendet, die jeweils für unterschiedliche Selektionsmarker kodieren und die eine ausreichend hohe Transformationsrate garantieren und diejenigen Mutanten selektiert, die neben einer erniedrigten Restriktionsbarriere nach wie vor eine hohe Transformationseffizienz aufweisen.

9. Verfahren gemäss Anspruch 8, dadurch gekennzeichnet, dass die Transformations zu Beginn der Anreicherung mindestens zwischen $10^6$ und $10^8$ Transformanten liefert.

10. Verfahren gemäss Anspruch 8, dadurch gekennzeichnet, dass die Transformation mit Hilfe einer Elektroporation durchgeführt wird.

11. Verfahren gemäss Anspruch 8, dadurch gekennzeichnet, dass man in den verschiedenen Transformations/Selektionsrunden effizient transformierbare Vektoren eingesetzt, die eine gezielte und selektive Auswahl der positiven Transformanten erlauben.

28

12. Verfahren gemäss Anspruch 8, dadurch gekennzeichnet, dass besagte Vektoren ein oder mehrere Markergene enthalten, welche der Wirtszelle eine Eigenschaft verleihen, die es erlauben, die mit dem Vektor transformierten Zellen zu erkennen und in der Folge zu selektionieren.

13. Verfahren gemäss Anspruch 12, dadurch gekennzeichnet, dass besagte Markergene
    (a) eine Antibiotikaresistenz kodieren;
    (b) ein Enzym kodieren, für das ein chromogenes Substrat vorhanden ist; oder
    (c) eine Resistenz gegen Schwermetalle vermitteln.

14. Verfahren gemäss Anspruch 8, dadurch gekennzeichnet, dass man im Zuge der Selektion geeigneter Mutanten zunächst Plasmid-freie Abkömmlinge herstellt und diese anschliessend mit einem Shuttlevektor transformiert, welcher bekanntermassen der Restriktion unterliegt.

15. Verfahren zur Klonierung von Genen oder sonstigen DNA Sequenzen in *Bacillus thuringiensis* und/oder *Bacillus cereus* unter Verwendung einer restriktionsdefizienten Mutanten gemäss einem der Ansprüche 1 bis 7, dadurch gekennzeichnet, dass man
    (a) besagte Gene oder DNA Sequenzen aus einer geeigneten Quelle isoliert oder aber diese synthetisiert;
    (b) die isolierten bzw. synthetisierten Gene oder DNA Sequenzen mit Expressionssignalen, die in *Bacillus thuringiensis* und/oder *Bacillus cereus* funktionsfähig sind und die homologen oder heterologen Ursprungs sein können in Bezug auf die verwendeten Gene oder DNA Sequenzen, operabel verknüpft;
    (c) die chimäre genetische Konstruktion gemäss Abschnitt (b) unter Verwendung geeigneter Vektoren in eine Restriktions-defiziente Mutante von *Bacillus thuringiensis* und/oder *Bacillus cereus* gemäss einem der Ansprüche 1 bis 6 transformiert; und
    (d) gegebenfalls ein entsprechendes Genprodukt exprimiert und, sofern gewünscht, isoliert.

16. Verfahren gemäss Anspruch 15, dadurch gekennzeichnet, dass man einen zusätzlichen Verfahrensschritt zwischenschaltet, der dadurch gekennzeichnet ist, dass man die Vektor-DNA zusammen mit einer spezifischen Methylase, die in der Lage ist eine oder mehrere Basen innerhalb der Erkennungssequenz einer wirtsspezifischen Restriktionsendonuklease zu methylieren, in einem geeigneten Reaktionsansatz *in vitro* inkubiert und anschliessend die nunmehr methylierte Vektor-DNA in eine restriktionsdefiziente Mutante von *B. thuringiensis* und/oder *B. cereus* transformiert.

17. Verfahren gemäss einem der Ansprüche 15 oder 16, dadurch gekennzeichnet, dass besagte Vektor-DNA natürlicherweise einer Restriktion in *B. thuringiensis* und /oder *B. cereus* unterliegt.

18. Verfahren gemäss einem der Ansprüche 15 oder 16, dadurch gekennzeichnet, dass besagte Vektoren aus *E. coli* oder *B. subtilis* stammen oder zumindest aus diesen isoliert wurden.

19. Verfahren zur Erstellung von Genbanken in *B. thuringiensis* und/oder *B. cereus*, dadurch gekennzeichnet, dass man
    (a) die Gesamt-DNA von *Bacillus thuringiensis* mechanisch oder mit Hilfe geeigneter Restriktionsenzyme in Fragmente zerlegt;
    (b) Fragmente geeigneter Grösse isoliert;
    (c) besagte Fragmente in einen geeigneten Vektor einspleisst;
    (d) restriktionsdefiziente *Bacillus thuringiensis* und/oder Bacillus cereus Zellen mit besagtem Vektor transformiert; und
    (e) aus den Transformanten mit Hilfe geeigneter Screeningverfahren diejenigen ausliest, die neue und gewünschte DNA Sequenzen enthalten.

20. Verfahren gemäss Anspruch 19, dadurch gekennzeichnet, dass man einen zusätzlichen Verfahrensschritt zwischenschaltet, der dadurch gekennzeichnet ist, dass dass man die Vektor-DNA zusammen mit einer spezifischen Methylase, die in der Lage ist eine oder mehrere Basen innerhalb der Erkennungssequenz einer wirtsspezifischen Restriktionsendonuklease zu methylieren, in einem geeigneten Reaktionsansatz *in vitro* inkubiert und anschliessend die methylierte Vektor-DNA in eine restriktionsdefiziente Mutante von *B. thuringiensis* und/oder *B. cereus* transformiert.

21. Verfahren gemäss einem der Ansprüche 19 oder 20, dadurch gekennzeichnet, dass es sich bei besagtem *Bacillus thuringiensis* um Stämme handelt, die ein vergleichbares RestriktionsModifikationssystem aufweisen wie der *Bacillus thuringiensis* Stamm HD1cryB.

**22.** Verfahren zur Reduktion von Restriktionsbarrieren bei *B. thuringiensis* und/oder *B. cereus*, dadurch gekennzeichnet, dass man eine restriktionsdefiziente Mutante von *B. thuringiensis* und/oder *B. cereus*, insbesondere aber die restriktions-negative Mutante *B. thuringiensis* HD1cryB Res9 verwendet.

**23.** Verfahren gemäss Ansprüch 22, dadurch gekennzeichnet, dass man eine restriktionsdefiziente Mutante von *B. thuringiensis* und/oder *B. cereus*, insbesondere aber die restriktions-negative Mutante *B. thuringiensis* HD1cryB Res9, in Kombination mit einer spezifischen Methylase verwendet, die unter Methylierung der eingeschleusten DNA diese vor Verdau durch *B.thuringiensis*- bzw. *B. cereus*-eigene Restriktionsenzyme schützt und somit die Effizienz des Verfahrens weiter erhöht.

**Patentansprüche für folgenden Vertragsstaat : ES**

**1.** Verfahren zur Herstellung einer Mutante von *B. thuringiensis* und/oder *B. cereus*, die eine partielle Defiziens innerhalb der inhärent im unmutierten Ausgangsstamm vorliegenden Restriktionsbarrieren aufweist und daher bei Verwendung von Vektor-DNA aus einem heterologen Zwischenwirt, die natürlicherweise einer Restriktion in *B. thuringiensis* und/oder *B. cereus* unterliegt, im Vergleich zu besagtem, nicht-mutierten Ausgangsstamm signifikant besser transformierbar ist, dadurch gekennzeichnet dass man Spontanmutanten mit verminderter Restriktionsbarriere anreichert, indem man mehrere Transformations- und Selektionsrunden hintereinanderschaltet, wobei man für die Transformation vorzugsweise Shuttlevektoren verwendet, die jeweils für unterschiedliche Selektionsmarker kodieren und die eine ausreichend hohe Transformationsrate garantieren und diejenigen Mutanten selektiert, die neben einer erniedrigten Restriktionsbarriere nach wie vor eine hohe Transformationseffizienz aufweisen.

**2.** Verfahren gemäss Anspruch 1, dadurch gekennzeichnet, dass die Transformations zu Beginn der Anreicherung mindestens zwischen $10^6$ und $10^8$ Transformanten liefert.

**3.** Verfahren gemäss Anspruch 1, dadurch gekennzeichnet, dass die Transformation mit Hilfe einer Elektroporation durchgeführt wird.

**4.** Verfahren gemäss Anspruch 1, dadurch gekennzeichnet, dass man in den verschiedenen Transformations/Selektionsrunden effizient transformierbare Vektoren eingesetzt, die eine gezielte und selektive Auswahl der positiven Transformanten erlauben.

**5.** Verfahren gemäss Anspruch 4, dadurch gekennzeichnet, dass besagte Vektoren ein oder mehrere Markergene enthalten, welche der Wirtszelle eine Eigenschaft verleihen, die es erlauben, die mit dem Vektor transformierten Zellen zu erkennen und in der Folge zu selektionieren.

**6.** Verfahren gemäss Anspruch 5, dadurch gekennzeichnet, dass besagte Markergene
(a) eine Antibiotikaresistenz kodieren;
(b) ein Enzym kodieren, für das ein chromogenes Subsaat vorhanden ist; oder
(c) eine Resistenz gegen Schwermetalle vermitteln.

**7.** Verfahren gemäss Anspruch 1, dadurch gekennzeichnet, dass man im Zuge der Selektion geeigneter Mutanten zunächst Plasmid-freie Abkömmlinge herstellt und diese anschliessend mit einem Shuttlevektor transformiert, welcher bekanntermassen der Restriktion unterliegt.

**8.** Verfahren gemäss Anspruch 1, dadurch gekennzeichnet,
dass besagte restriktionsdefiziente Mutante bei Verwendung von Vektor-DNA aus einem nicht-kompatiblen Zwischenwirt, die natürlicherweise einer Restriktion in *B. thuringiensis* und/oder *B. cereus* unterliegt, im Vergleich zum nicht-mutierten Ausgangsstamm signifikant besser transformierbar ist.

**9.** Verfahren gemäss Anspruch 8, dadurch gekennzeichnet, dass es sich bei besagtem nicht-kompatiblen Zwischenwirt um *E. coli* oder *Bacillus subtilis* handelt.

**10.** Verfahren gemäss Anspruch 4, dadurch gekennzeichnet, dass es sich bei besagtem unmutierten Ausgangsstamm um den *B. thuringiensis* Stamm HD1cryB handelt.

11. Verfahren gemäss Anspruch 1, dadurch gekennzeichnet, dass besagte Mutante eine im Vergleich zum nicht-mutierten Ausgangsstamm um den Faktor $\geqq 10^2$ bessere Transformierbarkeit aufweist.

12. Verfahren gemäss Anspruch 11, dadurch gekennzeichnet, dass besagte Mutante die folgenden, kennzeichnenden, taxonomischen Charakteristika aufweist:

| | |
|---|---|
| Stäbchen | |
| Breite /µm | 1,0-1,2 |
| Länge /µm | 3,0-5,0 |
| Beweglichkeit | + |
| Sporen | + |
| ellipsoid | + |
| rund | - |
| Sporangium angeschwollen | - |

| | |
|---|---|
| Gram Reaktion | + |
| Catalase | + |
| Anaerobes Wachstum | + |
| VP Reaktion | + |
| pH in VP-Medium | 4,7 |
| Maximale Temperatur | |
|    Wachstum positiv bei °C | 45 |
|    Wachstum negativ bei °C | 50 |
| Wachstum in | |
|    Medium pH 5.7 | + |
|    NaCl    5 % | + |
|        7 %   - | |
|       10 %   - | |
| Säure aus | |
|    Glucose | + |
|    L-Arabinose | - |
|    Xylose | - |
|    Mannit | - |
| Gas aus Glucose | - |
| Lecithinase | - |
| Hydrolyse von | |
|    Stärke | + |
|    Gelatine | + |
|    Casein | + |

Verwertung von

    Citrat                                                   +

    Propionat                                         -

Abbau von Tyrosin                            -

NO2 aus NO3                                +

Indol                                             -

Phenylalanindesaminase                  -

Arginindihydrolase                          +

ungewöhnliche Eigenschaft:
keine Lecithinase-Aktivität

13. Verfahren zur Klonierung von Genen oder sonstigen DNA Sequenzen in *Bacillus thuringiensis* und/oder *Bacillus cereus* unter Verwendung einer restriktionsdefizienten Mutanten, hergestellt nach einem Verfahren gemäss einem der Ansprüche 1 bis 12, dadurch gekennzeichnet, dass man
(a) besagte Gene oder DNA Sequenzen aus einer geeigneten Quelle isoliert oder aber diese synthetisiert;
(b) die isolierten bzw. synthetisierten Gene oder DNA Sequenzen mit Expressionssignalen, die in *Bacillus thuringiensis* und/oder *Bacillus cereus* funktionsfähig sind und die homologen oder heterologen Ursprungs sein können in Bezug auf die verwendeten Gene oder DNA Sequenzen, operabel verknüpft;
(c) die chimäre genetische Konstruktion gemäss Abschnitt (b) unter Verwendung geeigneter Vektoren in eine gemäss einem der Ansprüche 1 bis 12 hergestellte restriktionsdefiziente Mutante von *Bacillus thuringiensis* und/oder *Bacillus cereus* transformiert; und
(d) gegebenfalls ein entsprechendes Genprodukt exprimiert und, sofern gewünscht, isoliert.

14. Verfahren gemäss Anspruch 13, dadurch gekennzeichnet, dass man einen zusätzlichen Verfahrensschritt zwischenschaltet, der dadurch gekennzeichnet ist, dass man die Vektor-DNA zusammen mit einer spezifischen Methylase, die in der Lage ist eine oder mehrere Basen innerhalb der Erkennungssequenz einer wirtsspezifischen Restriktionsendonuklease zu methylieren, in einem geeigneten Reaktionsansatz *in vitro* inkubiert und anschliessend die nunmehr methylierte Vektor-DNA in eine restriktionsdefiziente Mutante von *B. thuringiensis* und/oder *B. cereus* transformiert.

15. Verfahren gemäss einem der Ansprüche 13 oder 14, dadurch gekennzeichnet, dass besagte Vektor-DNA natürlicherweise einer Restriktion in *B. thuringiensis* und /oder *B. cereus* unterliegt.

16. Verfahren gemäss einem der Ansprüche 13 oder 14, dadurch gekennzeichnet, dass besagte Vektoren aus *E. coli* oder *B. subtilis* stammen oder zumindest aus diesen isoliert wurden.

17. Verfahren zur Erstellung von Genbanken in *B. thuringiensis* und/oder *B. cereus*, dadurch gekennzeichnet, dass man
(a) die Gesamt-DNA von *Bacillus thuringiensis* mechanisch oder mit Hilfe geeigneter Restriktionsenzyme in Fragmente zerlegt;
(b) Fragmente geeigneter Grösse isoliert;
(c) besagte Fragmente in einen geeigneten Vektor einspleisst;
(d) gemäss einem der Ansprüche 1 bis 12 hergestellte, restriktionsdefiziente *Bacillus thuringiensis* und-

/oder *Bacillus cereus* Zellen mit besagtem Vektor transformiert; und

(e) aus den Transformanten mit Hilfe geeigneter Screeningverfahren diejenigen ausliest, die neue und gewünschte DNA Sequenzen enthalten.

18. Verfahren gemäss Ansprüch 17, dadurch gekennzeichnet, dass man einen zusätzlichen Verfahrensschritt zwischenschaltet, der dadurch gekennzeichnet ist, dass dass man die Vektor-DNA zusammen mit einer spezifischen Methylase, die in der Lage ist eine oder mehrere Basen innerhalb der Erkennungssequenz einer wirtsspezifischen Restriktionsendonuklease zu methylieren, in einem geeigneten Reaktionsansatz *in vitro* inkubiert und anschliessend die methylierte Vektor-DNA in eine restriktionsdefiziente Mutante von *B. thuringiensis* und/oder *B. cereus* transformiert.

19. Verfahren gemäss einem der Ansprüche 17 oder 18, dadurch gekennzeichnet, dass es sich bei besagtem *Bacillus thuringiensis* um Stämme handelt, die ein vergleichbares RestriktionsModifikationssystem aufweisen wie der *Bacillus thuringiensis* Stamm HD1cryB.

20. Verfahren zur Reduktion von Restriktionsbarrieren bei *B. thuringiensis* und/oder *B. cereus*, dadurch gekennzeichnet, dass man eine gemäss einem der Ansprüche 1 bis 12 hergestellte, restriktionsdefiziente Mutante von *B. thuringiensis* und/oder *B. cereus*, insbesondere aber die restriktions-negative Mutante *B. thuringiensis* HD1cryB Res9 verwendet.

21. Verfahren gemäss Anspruch 20, dadurch gekennzeichnet, dass man eine restriktionsdefiziente Mutante von *B. thuringiensis* und/oder *B. cereus*, insbesondere aber die restriktions-negative Mutante *B. thuringiensis* HD1cryB Res9, in Kombination mit einer spezifischen Methylase verwendet, die unter Methylierung der eingeschleusten DNA diese vor Verdau durch *B.thuringiensis*- bzw. *B. cereus*-eigene Restriktionsenzyme schützt und somit die Effizienz des Verfahrens weiter erhöht.